# EUROPÄISCHE PATENTANMELDUNG

(11) **EP 0 296 518 A1**
(43) Veröffentlichungstag der Anmeldung: **28.12.1988**
(21) Anmeldenummer: 88109775.2
(22) Anmeldetag: 20.06.1988
(51) Int. Cl.: C07D 405/14, C07D 401/12, C07D 409/12, C07D 417/12, C07D 405/12, A01N 43/653

(54) **Neue Phenylether-derivate als Mikrobizide, Verfahren zu deren Herstellung und deren Verwendung**

(30) Priorität: 22.06.1987 CH 2347/87
(71) Anmelder: CIBA-GEIGY AG, 4002 Basel (CH)
(72) Erfinder: Hubele, Adolf, Dr., CH-4312 Magden (CH)
(74) Vertreter: Zumstein, Fritz, Dr.

(57) **Zusammenfassung**

Es werden neue Phenylether-derivate der Formel I beschrieben 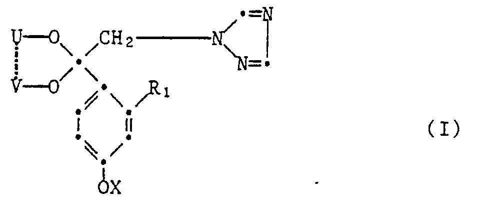 worin
X einen fünf- oder sechsgliedrigen ungesättigten heterocyclischen Rest mit einem, zwei oder drei Heteroatomen bedeutet, der unsubstituiert oder durch Halogen, Nitro, C₁-C₃-Alkyl, C₁-C₃-Alkoxy, C₁-C₃-Halogenal-
koxy oder Trifluormethyl mono-, di- oder trisubstituiert sein kann,
R₁ Wasserstoff, Halogen, Ci-C₃-Alkyl oder C₁-C₃-Halogenalkoxy bedeutet und
U und V unabhängig voneinander gegebenenfalls durch Halogen oder C₁-C₆-Alkoxy substituiertes C₁-C₁₂-Alkyl darstellen oder zusammen folgende Alkylenbrücken 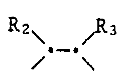 oder 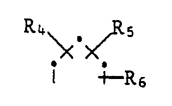 bilden, wobei
R₂ und R₃ unabhängig voneinander für Wasserstoff, Ci-C₆-Alkyl, ein-oder mehrfach durch Halogen substituiertes C₁-C₆-Alkyl, Phenyl, ein-oder mehrfach durch Halogen und/oder C₁-C₂-Alkyl substituiertes Phenyl oder die Gruppe -CH₂-Z-R₇ stehen oder worin R₂ und R₃ zusammen eine unsubstituierte oder durch C₁-C₄-Alkyl substituierte Tetramethylenbrücke bilden; und wobei
Z Sauerstoff oder Schwefel bedeutet;
R₇ für Wasserstoff, C₁-C₆-Alkyl, ein- oder mehrfach durch Halogen oder C₁-C₃-Alkoxy substituiertes C₁-C₆-Alkyl, oder für C₃-C₄-Alkenyl, 2-Propinyl, 3-Halo-2-propinyl, oder auch Phenyl oder Benzyl steht, deren aromatischer Ring unsubstituiert oder durch Halogen, C₁-C₃-Alkyl, C₁-C₃-Alkoxy, Nitro, und/oder CF₃ ein-oder mehrfach substituiert ist, steht;
R₄, R₅ und R₆ unabhängig voneinander Wasserstoff oder C₁-C₄-Alkyl bedeuten, wobei die Gesamtzahl der Kohlenstoffatome in R₄, Rs und R₆ die Zahl 6 nicht übersteigt;
unter Einschluss ihrer Säureadditionssalze und Metallkomplexe.

Ferner wird die Herstellung dieser Substanzen beschrieben, agrochemische Mittel, die als Wirkstoff eine dieser Verbindungen enthalten. Es wird die Herstellung dieser Mittel beschrieben und deren Verwendung. Sie lassen sich zur Bekämpfung von phytopathogenen Mikroorganismen einsetzen.

## Beschreibung

Die vorliegende Erfindung betrifft substituierte Phenylether-derivate der nachstehenden Formel I sowie deren Säureadditionssalze und Metallkomplexe. Sie betrifft ferner die Herstellung dieser Verbindungen sowie agrochemische Mittel, die als Wirkstoff mindestens eine der Verbindungen der Formel I enthalten, die Herstellung dieser agrochemischen Mittel und ihre Verwendung sowie ein Verfahren zur Bekämpfung oder Verhütung eines Befalls von Pflanzen durch phytopathogene Mikroorganismen.

Die erfindungsgemässen Arylphenylether-derivate haben die Formel I 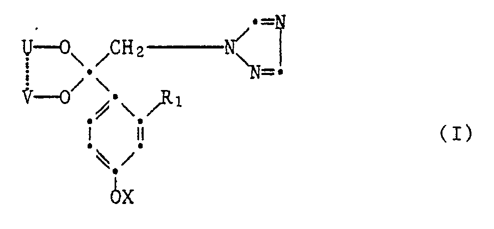 worin
X einen fünf- oder sechsgliedrigen ungesättigten heterocyclischen Rest mit einem, zwei oder drei Heteroatomen bedeutet, der unsubstituiert oder durch Halogen, Nitro, C₁-C₃-Alkyl, C₁-C₃-Alkoxy, C₁-C₃-Halogenal- koxy oder Trifluormethyl mono-, di- oder trisubstituiert sein kann,
R₁ Wasserstoff, Halogen, C₁-C₃-Alkyl oder C₁-C₃-Halogenalkoxy bedeutet und
U and V unabhängig voneinander gegebenenfalls durch Halogen oder C₁-C₆-Alkoxy substituiertes C₁-C₁₂-Alkyl darstellen oder zusammen folgende Alkylenbrücken 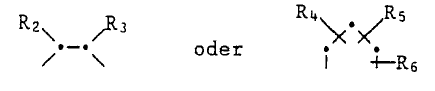 bilden, wobei
R₂ und R₃ unabhängig voneinander für Wasserstoff, C₁-C₆-Alkyl, ein-oder mehrfach durch Halogen substituiertes C₁-C₆-Alkyl, Phenyl, ein-oder mehrfach durch Halogen und/oder C₁-C₂-Alkyl substituiertes Phenyl oder die Gruppe -CH₂-Z-R₇ stehen oder worin R₂ und R₃ zusammen eine unsubstituierte oder durch C₁-C₄-Alkyl substituierte Tetramethylenbrücke bilden; und wobei
Z Sauerstoff oder Schwefel bedeutet;
R₇ für Wasserstoff, C₁-C₆-Alkyl, ein- oder mehrfach durch Halogen oder C₁-C₃-Alkoxy substituiertes C₁-C₆-Alkyl, oder für C₃-C₄-Alkenyl, 2-Propinyl, 3-Halo-2-propinyl, oder auch Phenyl oder Benzyl steht, deren aromatischer Ring unsubstituiert oder durch Halogen, C₁-C₃-Alkyl, C₁-C₃-Alkoxy, Nitro, und/oder Trifluormethyl ein- oder mehrfach substituiert ist;
R₄, Rs und R₆ unabhängig voneinander Wasserstoff oder C₁-C₄-Alkyl bedeuten, wobei die Gesamtzahl der Kohlenstoffatome in R₄, Rₛ und R₆ die Zahl 6 nicht übersteigt;
unter Einschluss ihrer Säureadditionssalze und Metallkomplexe.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl sowie ihre Isomeren wie z.B. Isopropyl, Isobutyl, tert.-Butyl, sek.-Butyl, Isopentyl usw.. Alkenyl steht z.B. für Propenyl-(1), Allyl, Butenyl-(1), Butenyl-(2) oder Butenyl-(3). Halogen soll hier und im folgenden Fluor, Chlor, Brom oder Jod, vorzugsweise Fluor, Chlor oder Brom, bedeuten.

Die Erfindung betrifft sowohl die freien Verbindungen der Formel I, als auch deren Additionssalze mit anorganischen und organischen Säuren, ebenso deren Komplexe mit Metallsalzen.

Erfindungsgemässe Salze sind insbesondere Additionssalze mit nach Massgabe des Einsatzzweckes physiologisch unbedenklichen anorganischen oder organischen Säuren, wie beispielsweise Halogenwasserstoffensäuren, z.B. Chlor-, Brom- oder Jodwasserstoffsäure, Schwefelsäure, Phosphorsäure, phosphorige Säure, Salpetersäure, gegebenenfalls halogenierte Fettsäuren, wie Essigsäure, Trichloressigsäure und Oxalsäure, oder Sulfonsäuren, wie Benzolsulfonsäure und Methansulfonsäure oder auch Additionssalze mit geeigneten Salzen, wie beispielsweise Calciumchlorid.

Metallkomplexe der Formel I bestehen aus dem zugrundeliegenden organischen Molekül und einem anorganischen oder organischen Metallsalz, z.B. den Halogeniden, Nitraten, Sulfaten, Phosphaten, Tartraten usw. des Kupfers, Mangans, Eisens, Zinks und anderer Metalle. Dabei können die Metallkationen in den verschiedenen ihnen zukommenden Wertigkeiten vorliegen.

Die Verbindungen der Formel I sind bei Raumtemperatur stabile Oele, Harze oder Feststoffe, die sich durch sehr wertvolle physiologische, wie mikrobizide, beispielsweise pflanzenfungizide Eigenschaften auszeichnen. Sie lassen sich daher einerseits auf Agrarsektor oder verwandten Gebieten zur Bekämpfung von phytopathogenen Mikroorganismen einsetzen.

Eine für den Einsatz im Pflanzenschutz wichtige Gruppe von Mikrobiziden besteht aus Verbindungen der Formel I, worin
X einen unsubstituierten oder durch Methoxy, Halogen, Nitro oder Trifluormethyl mono- oder disubstituierten 2-Pyridinylrest, den 2-Thienylrest, den 2-Thiazolylrest oder den 4-Nitro-2-thiazolylrest, den Pyrazinylrest, den 2-Pyrimidinylrest oder den 4,5,6-Trichlor-2-pyrimidinylrest,
R₁ Wasserstoff, Halogen, Methyl, Aethyl oder Difluormethoxy bedeuten
und U und V unabhängig voneinander unsubstituierte oder durch Chlor oder Trifluormethyl substituierte C,-C₁₂-Alkyl darstellen oder zusammen folgende Alkylenbrücken 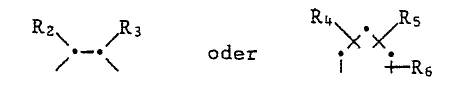 bilden, wobei
R₂ und R₃ unabhängig voneinander für Wasserstoff, Phenyl, durch Halogen substituiertes Phenyl, unsubstituiertes oder durch Halogen, C₁-C₃-Alkoxy, C₁-C₂-Halogenalkoxy, Allyloxy, Propargyloxy, substituiertes C,-C₃-Alkyl stehen.

Aus dieser Gruppe wird jene Gruppe von landwirtschaftlich nutzbaren Mikrobiziden bevorzugt, in welchen
X einen unsubstituierten oder durch Fluor, Chlor, Brom, Nitro oder Trifluormethyl mono- oder disubstituierten 2-Pyridinylrest, den 2-Thienylrest, den 2-Thiazolylrest, den Pyrazinylrest, den 2-Pyrimidinylrest oder den 4,5,6-Trichlor-2-pyrimidinylrest,
R₁ Wasserstoff, Chlor, Methyl oder Difluormethoxy bedeuten und U und V Methyl, Aethyl, Propyl darstellen oder zusammen folgende Alkylenbrücken 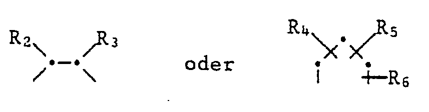 bilden, wobei
R₂ und R₃ unabhängig voneinander für Wasserstoff, C₁-C₃-Alkyl, Methoxymethyl, Aethoxymethyl oder Trifluoraethoxymethyl,
R₄ für Methyl, Rₛ für Wasserstoff oder Methyl und R₆ für Wasserstoff stehen.

Von den oben definierten Verbindungen werden jene insbesondere bevorzugt, bei welchen
X einen unsubstituierten oder einen durch Chlor, Brom oder Trifluormethyl mono- oder disubstituierten 2-Pyridinylrest oder den 2-Pyrazinylrest,
R₁ Methyl oder Chlor,
U und V zusammen den Methyl-äthylenrest, den Aethyl-äthylenrest oder den n-Propyl-äthylenrest bedeuten.

Aus dieser, letztgenannten Gruppe sind folgende Verbindungen hervorzuheben:
2-[p-(Pyridin-2-yloxy)-2'-methylphenyl]-2-[1 H-1,2,4-triazolylmethyl]-4-äthyl-1,3-dioxolan,
2-[p-(5-Chlor-3-fluorpyridin-2-yloxy)-2-chlorphenyl]-2-[1H-1,2,4-triazolylmethyl]-4-methyl-1,3-dioxolan,
2-[p-(5-Chlor-3-fluorpyridin-2-yloxy)-2-methylphenyl]-2-[1H-1,2,4-triazolylmethyl]-4-methyl-1,3-dioxolan,
2-[p-(5-Trifluormethylpyridin-2-yloxy)-2-methylphenyl]-2-[1H-1,2,4-triazolylmethyl]-4-methyl-1,3-dioxolan,
2-[p-(5-Brompyridin-2-yloxy)-2-methylphenyl]-2-[1H-1,2,4-triazolylmethyl]-4-methyl-1,3-dioxolan,
2-[p-(5-Chlorpyridin-2-yloxy)-2-methylphenyl]-2-[1H-1,2,4-triazolylmethyl]-4-äthyl-1,3-dioxolan,
2-[p-(5-Brompyridin-2-yloxy)-2-methylphenyl]-2-[1H-1,2,4-triazolylmethyl]-4-äthyl-1,3-dioxolan,
2-[p-(Pyridin-2-yloxy)-2-chlorphenyl]-2-[1H-1,2,4-triazolylmethyl]-4-äthyl-1,3-dioxolan,
2-[p-(5-Trifluormethylpyridin-2-yloxy)-2-chlorphenyl]-2-(1H-1,2,4-triazolylmethyl]-4-methyl-1,3-dioxolan,
2-[p-(5-Trifluormethylpyridin-2-yloxy)-2-chlorphenyl]-2-[1H-1,2,4-triazolylmethyl]-4-äthyl-1,3-dioxolan,
2-[p-(Pyrazin-2-yloxy)-2-chlorphenyl]-2-[1H-1,2,4-triazolylmethyl]-4-äthyl-1,3-dioxolan.

Die Verbindungen der Formel I können hergestellt werden, indem man
A) Verbindungen der Formeln II und III 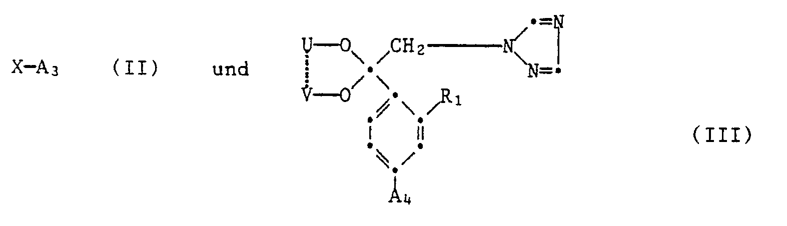 worin einer der Reste A₃ und A₄ eine Gruppe -O-Me, in der Me Wasserstoff oder vorzugsweise ein Metallkation ist, und der andere einen, gegen einen definitionsgemäss substituierten Hydroxylrest austauschbaren Rest bedeutet, miteinander kondensiert oder
B) eine Verbindung der Formel IV 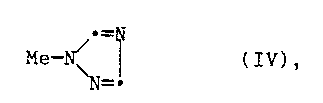 worin Me Wasserstoff oder ein Metallkation darstellt, mit einer Verbindung der Formel V 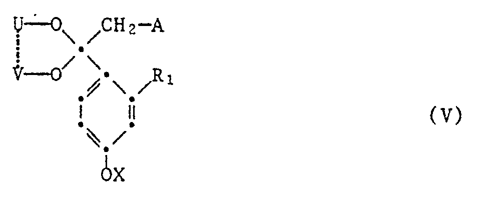 worin A eine nukleofuge Abgangsgruppe bedeutet, kondensiert oder
C) in einer Verbindung der Formel VI 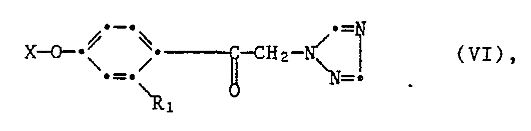 die Carbonylgruppe in eine Gruppe der Formel VII 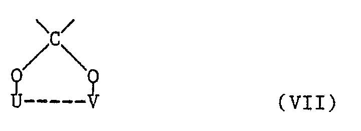 überführt, oder
D) zur Hestellung von Verbindungen der Formel I, worin U und V gemeinsam eine Gruppe der Formel -CH₂-CH(CH₂ZR₇)- darstellen und R7 einen von Wasserstoff verschiedenen Rest R₇ bedeutet, Verbindungen der Formeln VIII und IX 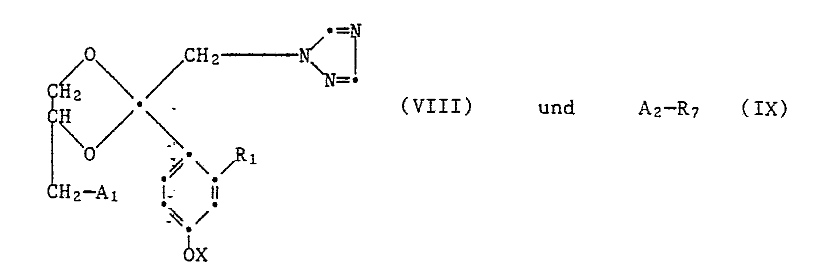 worin einer der Reste A₁ und A₂ gegebenenfalls in Salzform vorliegendes Hydroxy oder Mercapto, z.B. der Formel -Z-Me, und der andere eine nukleofuge Abgangsgruppe A bedeutet oder sowohl A₁ als auch A₂ Hydroxygruppen darstellen, miteinander kondensiert, und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhältliche freie Verbindung bzw. ein verfahrensgemäss erhältliches Säureadditionssalz in einen Metallkomplex überführt.

Metallkationen Me sind dabei beispielsweise Alkalimetall-, z.B. Lithium-, Natrium- oder Kaliumkationen, oder Erdalkalimetall, z.B. Magnesium-, Calcium-, Strontium- oder Bariumkationen.

Von den angeführten Herstellungsverfahren wird die Variante A) bevorzugt. Dabei geht man von Verbindungen der Formeln II und III aus, worin A₃ eine Gruppe -OMe and A₄ eine nukleofuge Abgangsgruppe ist oder bevorzugt, A₃ die nukleofuge Abgangsgruppe darstellt und A₄ die Gruppe -OMe bildet.

Nukleofuge Abgangsgruppen sind beispielsweise reaktionsfähige veresterte Hydroxygruppen, wie die mit einer Halogenwasserstoffsäure, z.B. mit Fluor-, Chlor-, Brom- oder Jodwasserstoffsäure, oder die mit einer Niederalkansulfonsäure, oder die mit einer gegebenenfalls substituierten Benzol- oder Halogensulfonsäure; beispielsweise mit Methan-, Ethan-, Benzol-, p-Toluol- oder Fluorsulfonsäure veresterte Hydroxygruppen.

Die Substituenten Ri, U, V und Ar haben die unter Formel I angegebenen Bedeutungen; Me steht bevorzugt für Wasserstoff. Die Reaktion wird bevorzugt in einem relativ polaren, jedoch reaktionsinerten organischen Lösungsmittel durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol u.a. verwendet werden.

Die Umsetzung eines Azols der Formel IV 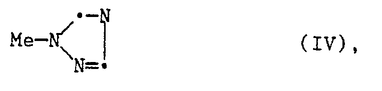 worin Me bevorzugt ein Metallatom, insbesondere ein Alkalimetallatom darstellt, nach Variante B), mit einer Verbindung der Formel V 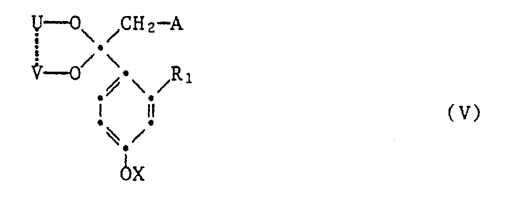 worin X. Ri, U and V die unter Formel I angegebenen Bedeutungen haben wird, wie die Umsetzung nach Variante A), bevorzugt in einem relativ polaren, jedoch reaktionsinerten organischen Lösungsmittel durchgeführt.

Bedeutet A Chlor oder Brom, so kann man zweckmässig Alkalijodid (wie NaJ oder KJ) zur Beschleunigung der Reaktion zusetzen. Erhöhte Temperaturen von 0 bis 220 °C, bevorzugt 120-270 C, sind vorteilhaft. Zweckmässig wird das Reaktionsgemisch unter Rückfluss erhitzt.

In den Fällen, in denen in Formel IV Me für Wasserstoff steht, wird das Verfahren in Gegenwart einer Base durchgeführt. Beispiele solcher Basen sind anorganische Basen wie die Oxide, Hydroxide, Hydride,Carbonate und Hydrogencarbonate von Alkali und Erdalkalimetallen, wie z.B. tert. Amine wie Triethylamin, Triethylendiamin, Piperidin, Pyridin, 4-Dimethylaminopyridin, 4-Pyrrolidylpyridin usw.

Bei dieser und bei den anderen hier beschriebenen Herstellungsvarianten können die Zwischen- und Endprodukte aus dem Reaktionsmedium isoliert und, falls erwünscht, auf eine der allgemein üblichen Methoden gereinigt werden, z.B. durch Extraktion, Kristallisation, Chromatographie, Destillation usw.

Die Ueberführung der Carbonylgruppe in Verbindungen der Formel VI in die Gruppe der Formel VII erfolgt durch Umsetzung mit einem Orthocarbonsäure-tri-C₁-C₁₂-alkylester, dessen C₁-C₁₂-Alkylgruppen gegebenenfalls durch Halogen oder Ci-C₆-Alkoxy substituiert sind, oder in Gegenwart einer Säure mit mindestens 2 Mol eines einwertigen Alkohols der Formel U-OH (VII), wobei Verbindungen der Formel I erhalten werden, worin U und V gleiche, gegebenenfalls substituierte C₁-C₁₂-Alkylgruppen bedeuten, oder durch Umsetzung mit einem Diol der Formel Vllb
HO-U-----V-OH (Vllb), wobei Verbindungen der Formel 1 erhalten werden, worin U und V gemeinsam eine der eingangs definierten Alkylenbrücken darstellen. Dabei haben X, Ri, U und V die unter Formel I angegebenen Bedeutungen.

Diese Ketalisierungsreaktion kann analog zu bereits bekannten Ketalisierungs-Reaktionen durchgeführt werden, beispielsweise analog zur Herstellung von 2-Brommethyl-2,4-diphenyl-1,3-dioxolan [Synthesis, 1974 (I), 23].

Bei der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure, vorteilhaft sein kann. Verwendbare organische Lösungsmittel sind in diesem Fall z.B. aromatische Kohlenwasserstoffe, wie Benzol, Toluol, Xylol, usw., gesättigte Kohlenwasserstoffe, wie n-Hexan oder gesättigte halogenierte Kohlenwasserstoffe wie z.B. 1,1,1-Trichlorethan.

Es sind auch weitere Wege der Ketalisierung durchführbar, z.B. indem man ein Keton VI, das mit einem von Alkanolen bzw. Diolen der Formeln Vlla bzw. Vllb verschiedenen Alkohol ketalisiert ist, umsetzt und dieses durch Reaktion mit überschüssigem Alkanol Vlla bzw. dem Diol Vllb zu (I) umketalisiert. Das Ausgangsprodukt ist z.B. gemäss Verfahrensvariante A) zugänglich.

Die Herstellung von Verbindungen der Formel I, worin Substituenten U und V gemäss Variante D) zusammen für -CH₂-CH(CH₂ZR₇)- stehen, erfolgt beispielsweise durch Reaktion einer Verbindung der Formel VIII mit einer Verbindung der Formel IX, worin A₁ eine Gruppe -ZH und A₂ eine Gruppe A bedeutet. Die Reaktion wird bevorzugt in reaktions-inerten organischen Lösungsmitteln durchgeführt. Es eignen sich hierzu z.B. N,N-Dimethylformamid, N,N-Dimethylacetamid, Hexamethyphosphortriamid, Dimethylsulfoxid, 4-Methyl-3-pentanon usw. Auch Gemische mit anderen reaktionsinerten Lösungsmitteln, z.B. mit aromatischen Kohlenwasserstoffen wie Benzol, Toluol, Xylol usw. können verwendet werden. In manchen Fällen kann es sich als vorteilhaft erweisen, zur Beschleunigung der Reaktionsgeschwindigkeit in Gegenwart einer Base zu arbeiten. Solche Basen sind z.B. Alkalimetallhydride oder Alkalimetallcarbonate. In gewissen Fällen kann es auch von Vorteil sein, dass man die Verbindung VIII zuerst auf bekannte Art in ein geeignetes Metall-Salz überführt.

Dies geschieht vorzugsweise durch Reaktion von VIII mit einer Na-Verbindung, z.B. Natriumhydrid, Natriumhydroxid usw.. Anschliessend wird dieses Salz von VIII mit der Verbindung der Formel IX umgesetzt. Zur Erhöhung der Reaktionsgeschwindigkeit kann in manchen Fällen auch bei erhöhter Temperatur, vorzugsweise 80°C bis 130 °C, bzw. am Siedepunkt des Lösungsmittels gearbeitet werden.

In analoger Weise kann man auch Verbindungen der Formeln VIII und IX, worin A₁ eine Gruppe A und A₂ eine Gruppe -ZH ist, umsetzen.

Bei der zu Produkten der Formel I, worin Z Sauerstoff ist, führenden Kondensations-Reaktion von Verbindungen der Formel VIII und IX, worin A₁ und A₂ Hydroxy darstellen, können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder Alkohol HO-R₇ selbst in Frage. Bei dieser Reaktion arbeitet man zweckmässigerweise in Gegenwart einer starken Säure, z.B. p-Toluolsulfonsäure.

Verfahrensgemäss erhältliche Verbindungen können nach an sich bekannten Methoden in andere Verbindungen der Formel I überführt werden.

So kann man beispielsweise verfahrensgemäss erhältliche Verbindungen zu anderen Verbindungen der Formel I umketalisieren. Beispielsweise kann man in Verbindungen der Formel I worin U und V gleiche, gegebenenfalls substituierte, C₁-C₁₂-Alkylreste U darstellen, durch Umsetzung mit 1 Mol eines anderen, gegebenenfalls substituierten C₁-C₁₂-Alkanols der Formel VII-OH (VIIc), eine Gruppe U durch eine Gruppe VII oder durch Umsetzung mit einem Diol der Formel VIIb beide Gruppen U durch einen zweiwertigen Rest ersetzen. Die Umketalisierung erfolgt in üblicher Weise, beispielsweise in Gegenwart eines sauren Kondensationsmittels, wie einer Mineral-, Sulfon- oder starken Carbonsäure, z.B. von Chlor- oder Bromwasserstoffsäure, Schwefelsäure, p-Toluolsulfonsäure oder Trifluoressigsäure, vortelhaft unter destillativer bzw. azeotrop-destillativer Entfernung leichtflüchtiger Reaktionsprodukte.

Ferner kann man in die cyclischen Arylteile verfahrensgemäss erhältlicher Verbindungen gegebenenfalls zusätzliche Substituenten in den Rest X und/oder die Gruppen R₁ einführen. So kann man z.B. durch Umsetzung mit einem Halogen in Gegenwart einer Lewissäure, wie einem Eisen-, Zink-, Bor- oder Antimonhalogenides, oder durch Behandlung mit N-Chlorsuccinimid, Halogen einführen.

Ferner kann man Nitrogruppen, z.B. mittels geeigneter komplexer Hydride, z.B. mit Lithiumaluminiumhydrid, zu Aminen reduzieren, diese, z.B. mittels salpetriger Säure, diazotieren und die gebildete Diazoniumgruppe in üblicher Weise durch Halogen oder Alkoxy ersetzen. Ebenso kann man Halogen durch Umsetzung mit einer Alkylmetallverbindung, z.B. mit einem Alkyllithium oder Alkylmagnesiumhalogenid, durch Alkyl ersetzen.

Werden die Verbindungen der Formel I als Basen erhalten, dann lassen sie sich mit anorganischen oder organischen Säuren in entsprechende Salze oder mit vorzugsweise äquimolaren Mengen von Metallsalzen in Metallkomplexe der Formel 1 überführen. Umgekehrt lassen sich Salze der Formel I beispielsweise durch Reaktion mit Alkali(hydrogen)carbonat oder Alkalihydroxid in die freien Basen der Formel I überführen.

Die Zwischenprodukte III, V und VI sind nach den üblichen Methoden der präparativen organischen Chemie herstellbar. So kann beispielsweise das nach Variante A) eingesetzte Phenolderivat III (A₄ = -OH) durch katalytische Hydrogenolyse des durch Umsetzung des Triazols IV mit dem bromierten Ketal der Formel X 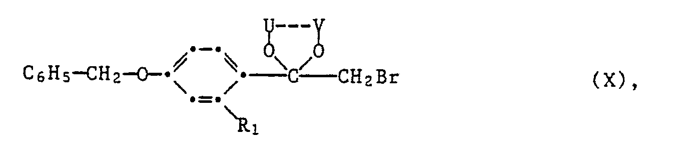 erhältlichen Benzyläthers III (A₄ = C₆H₅CH₂O-) dargestellt werden.

Das bromierte Ketal (X) ist ausgehend aus dem Acetophenonderivat der Formel XI 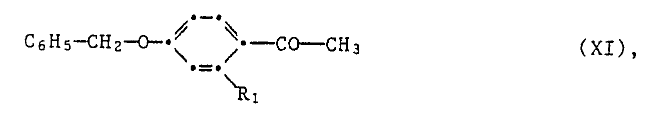 durch Seitenkettenbromierung und Acetalisierung herstellbar, wobei die Reihenfolge dieser Operationen variierbar ist.

Das nach Variante B) eingesetzte Aether der Formel V kann, falls A = Br ist, durch Bromierung und Acetalisierung des Aethers der Formel XII 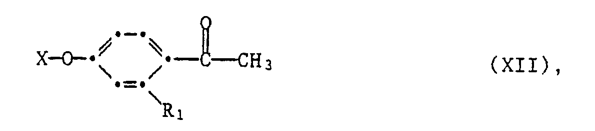 hergestellt werden, wobei die Reihenfolge dieser Operationen ebenfalls variierbar ist.

Der Aether der Formel XII ist durch Umsetzung des Halogenids XIII mit dem Phenol XIV erhältlich:

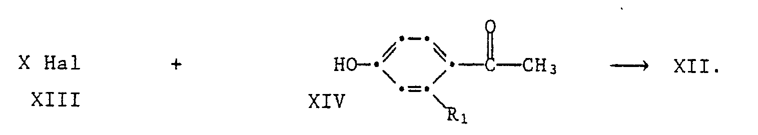

Der nach Variante C) eingesetzte Aether der Formel VI ist durch Kondensation des Bromketons der Formel XV mit dem Triazol IV herstellbar.

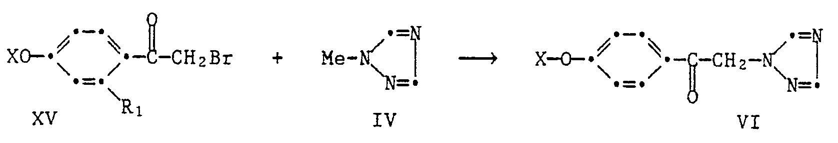

Die Ausgangsprodukte II, IV, XIV und die zur Acetalisierung eingesetzten Alkohole und Glykole sind bekannte Chemikalien.

Die beschriebenen Herstellunsvarianten sind Bestandteil der Erfindung.

Bei allen beschriebenen Ketalisierungs-Reaktionen eines Ketons mit einem substituierten α,β- oder α,γ,-Diol entstehen vorwiegend Gemische von Diastereomeren des resultierenden Ketals. Entsprechend bilden sich aus den Ausgangsketonen im allgemeinen Diastereomerengemische der Endprodukte I. Die Verbindungen der Formel I können beispielsweise in nachfolgenden beiden diastereomeren Formen vorliegen:

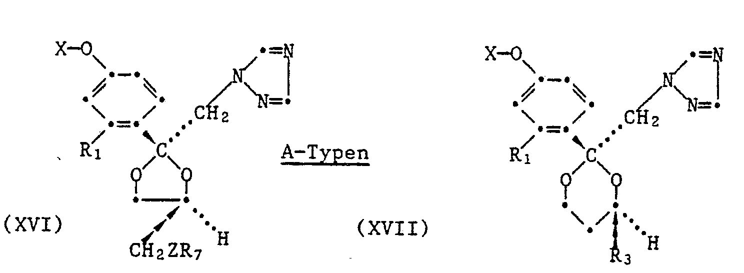

Die Konfiguration vom Typ A soll hier und im folgenden als das "trans"-Isomere oder "trans-Racemat" bezeichnet werden.

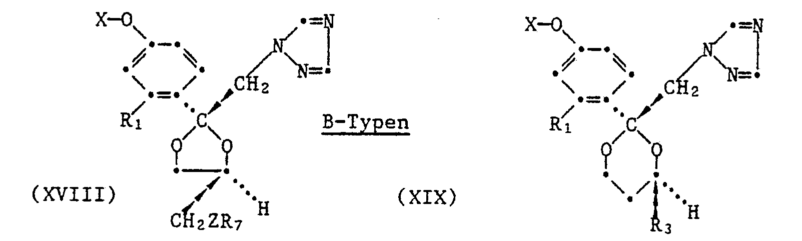

Die Konfiguration vom Typ B soll entsprechend als das "cis"-Isomere oder "cis-Racemat" bezeichnet werden.

Die Symbole in den räumlich wiedergegebenen Strukturen sollen folgende Bedeutungen haben: ... = hinter = in = vor der Zeichenebene..

Die Trennung der beiden Diastereomeren kann beispielsweise durch fraktionierte Kristallisation oder durch Chromatographie (Dünn-, Dickschicht-, Säulenchromatographie, Flüssigkeitshochdruckchromatographie usw.) erfolgen. Die Herstellung der vier optisch reinen Isomeren ist ebenfalls möglich. Sowohl die vier Isomeren, als auch die beiden "trans- und cis"-Racemate zeigen unterschiedliche biologische Wirkungen. Im allgemeinen werden für praktische Zwecke die bei der Herstellung anfallenden Diastereomerengemische als solche verwendet.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helmintosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel 1 bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln. Kirschen, Erd-, Hirn- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel 1 werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide. Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Bei allen Formulierungen können geeignete Träger und Zusätze fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der-Wirkstoffe mit Streckmittein, wie z.B.- mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylchlorin-Mischungen. Synthetische Phospholipide sind z.B. Dioctanoylphosphatidylcholin und Dipalmitoylphophatidylcholin.

Als oberflächenaktive Verbindugen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C) 0-022), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäureguppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome in Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte. Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:
"Mc Cutcheon's Detergents and Emulsifiers Annual" BC Publishing Corp., Ringwood New Jersey, 1981;
Helmut Stache "Tensid-Taschenbuch" Carl Hanser-Verlag München/Wien 1981.

M. and J. Ash. "Encyclopedia of Surfactants", Vol. 1-111, Chemical Publishing Co., New York, 1980-1981.

Die agrochemischen Zubereitungen enthalten in der Regel 0,1 bis 99%, insbesondere 0,1 bis 95% Wirkstoff der Formel I, 99,9 bis 1 %, insbesondere 99,8 bis 5% eines festen oder flüssigen Zusatzstoffes und 0 bis 25%, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht.

### Herstellungsbeispiele

Beispiel H1: Herstellung von 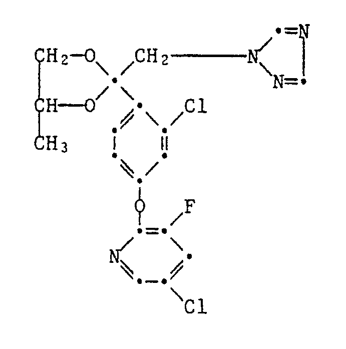 Verb. Nr. 1.3

### 2-[p-(5-Chlor-3-fluorpyridin-2-yloxy)-2'-chlorphenyl]-2-[1H-1,2,4-triazolylmethyl]-4-methyl-1,3-dioxolan

Zu einer Lösung von 4,5 g (15,2 mMol) 2-(4-Hydroxy-2-chlorphenyl)-2-(lH-1,2,4-triazolylmethyl)-4-methyl-1,3-dioxolan in 30 ml absolutem Methanol werden bei Raumtemperatur 2,88 g (16 mMol) 30%iges Natriummethylat in 10 ml absolutem Methanol zugetropft, eine halbe Stunde bei 60°C gerührt, das Methanol am Rotationsverdampfer entfernt und das kristalline Natriumsalz mit 40 ml absolutem Dimethylformamid versetzt. Zu diesem Gemisch werden 2,5 g (16,7 mMol) 5-Chlor-2,3-difluorpyridin in 10 ml absolutem Dimethylformamid innerhalb von 10 Minuten zugetropft, mit 0,3 g katalytisch wirkendem Kaliumjodid versetzt und 16 Stunden bei 100° C gerührt. Nach dem Abkühlen auf Raumtemperatur wird das Reaktionsgemisch auf 150 ml Wasser gegossen, dreimal mit je 50 ml Ethylacetat extrahiert, die vereinigten organischen Phasen zweimal mit je 50 ml Wasser gewaschen, über Natriumsulfat getrocknet, filtriert und das Lösungsmittel verdampft. Der hellbraune ölige Rückstand wird über eine 30 cm lange Kieselgelsäule mittels ChloroformiDiethylether (1:1) chromatographiert. Nach Verdampfen des Laufmittels wird ein gelbes Oel erhalten; n_{D}²⁴ : 1,5794.

### Beispiel H2: Herstellung des Zwischenproduktes

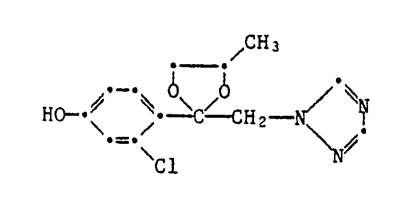

### 2-(4-Hydroxy-2-chlorphenyl)-2-(1 H-1,2,4-triazolylmethyl)-4-methyl-1,3-dioxolan

48,2 g (0.11 Mol) 2-(4-Benzyloxy-2-chlorphenyl)-2-(1H-1,2,4-triazolylmethyl)-4-methyl-1,3-dioxolan werden in 15%iger Aethanol-Lösung in Gegenwart von 5 % Palladium-Kohle bei Raumtemperatur hydriert, vom Katalysator abfiltriert und das Lösungsmittel verdampft. Nach dem Umkristallisieren aus Toluol/Petrolether (50-70°C) wird ein weisses Kristallpulver erhalten; Smp. 201-203°C.

Auf analoge Weise lassen sich auch die nachfolgenden Endprodukte (als Diastereomerengemische) mit unterschiedlichen Mischungsverhältnissen der Formel I herstellen:

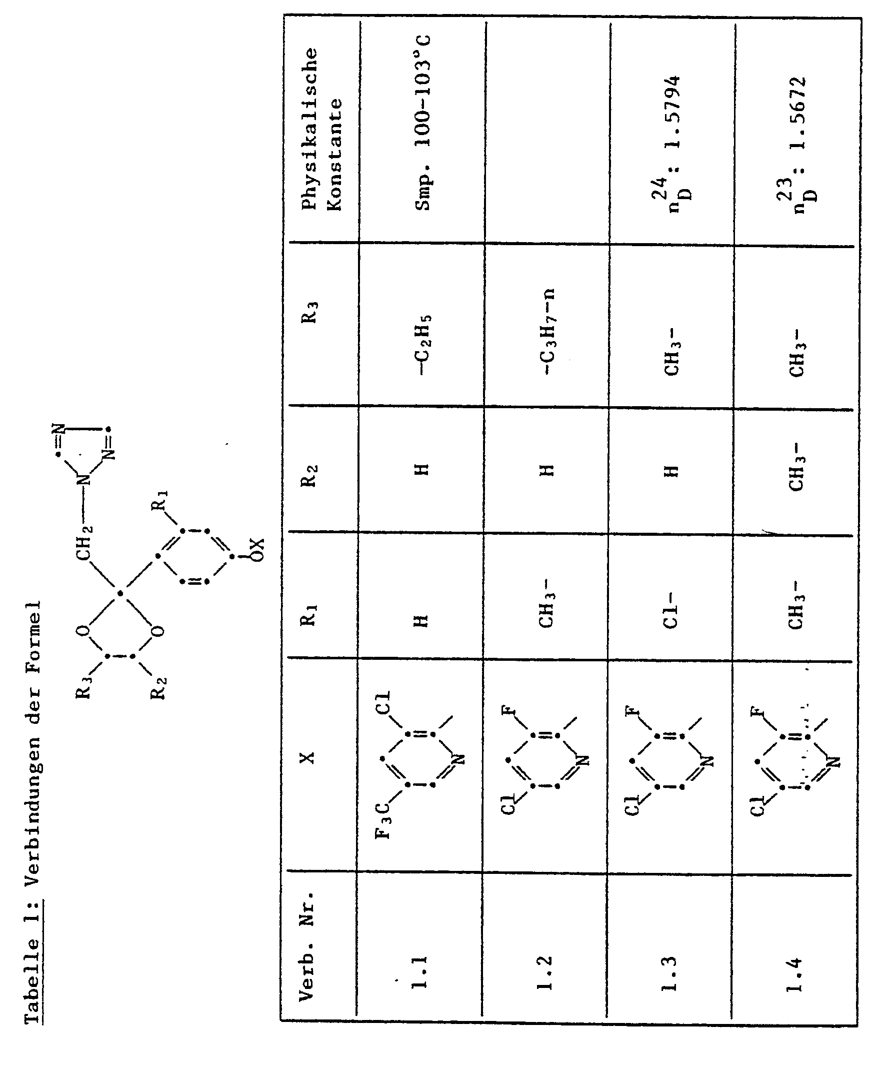

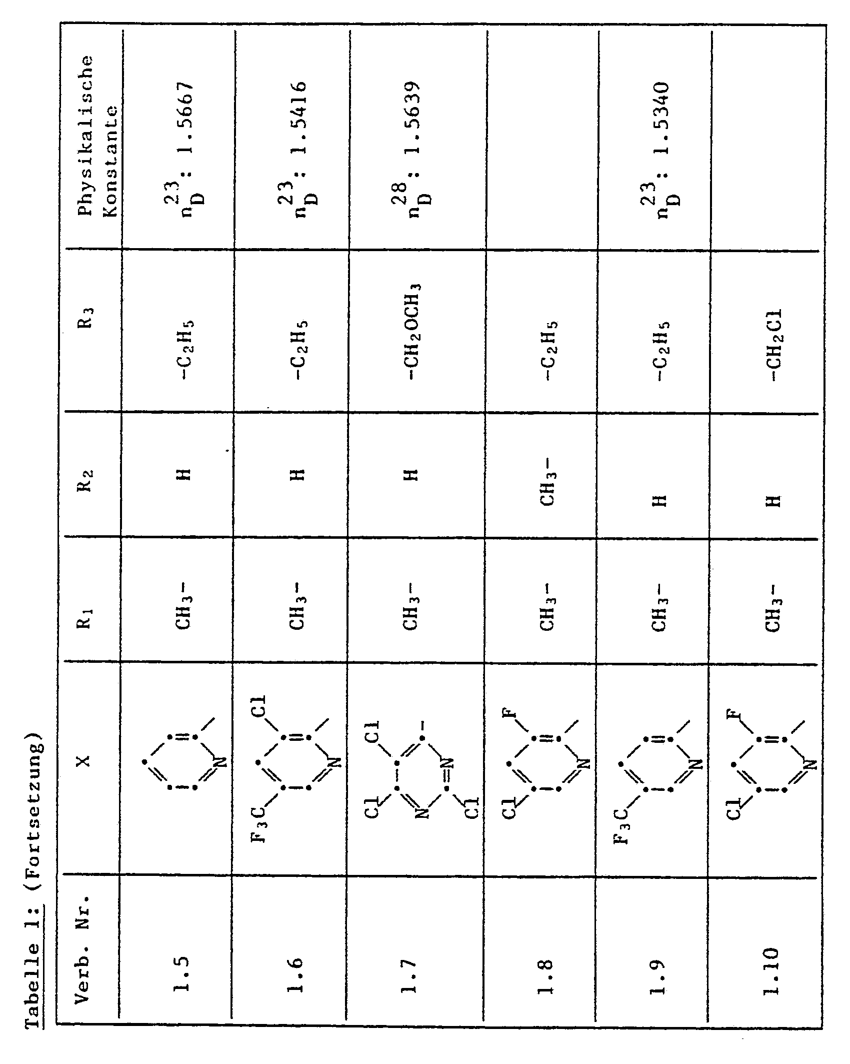

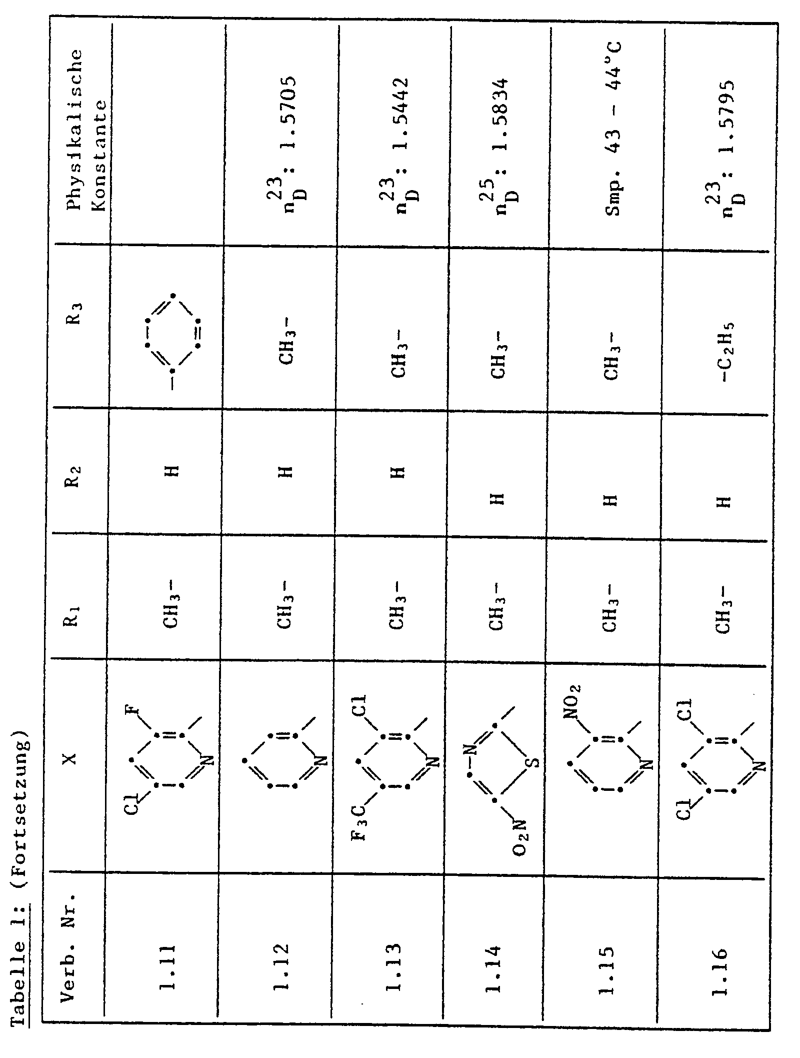

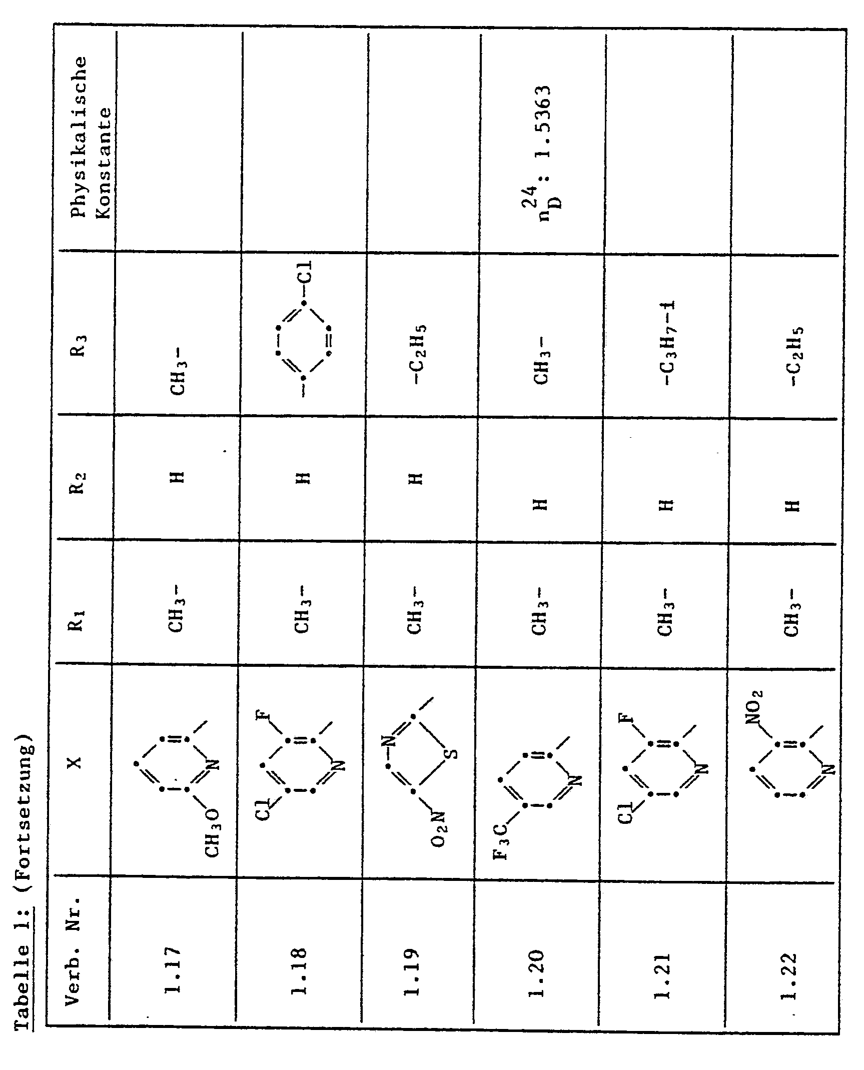

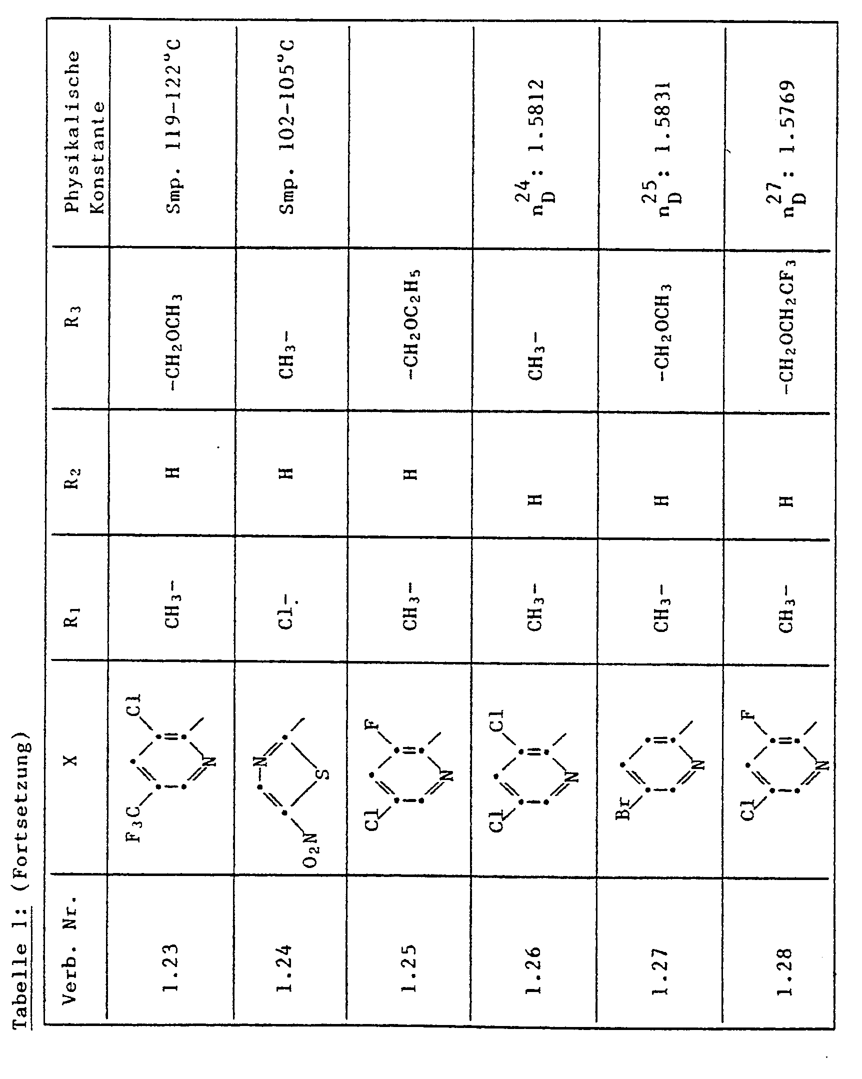

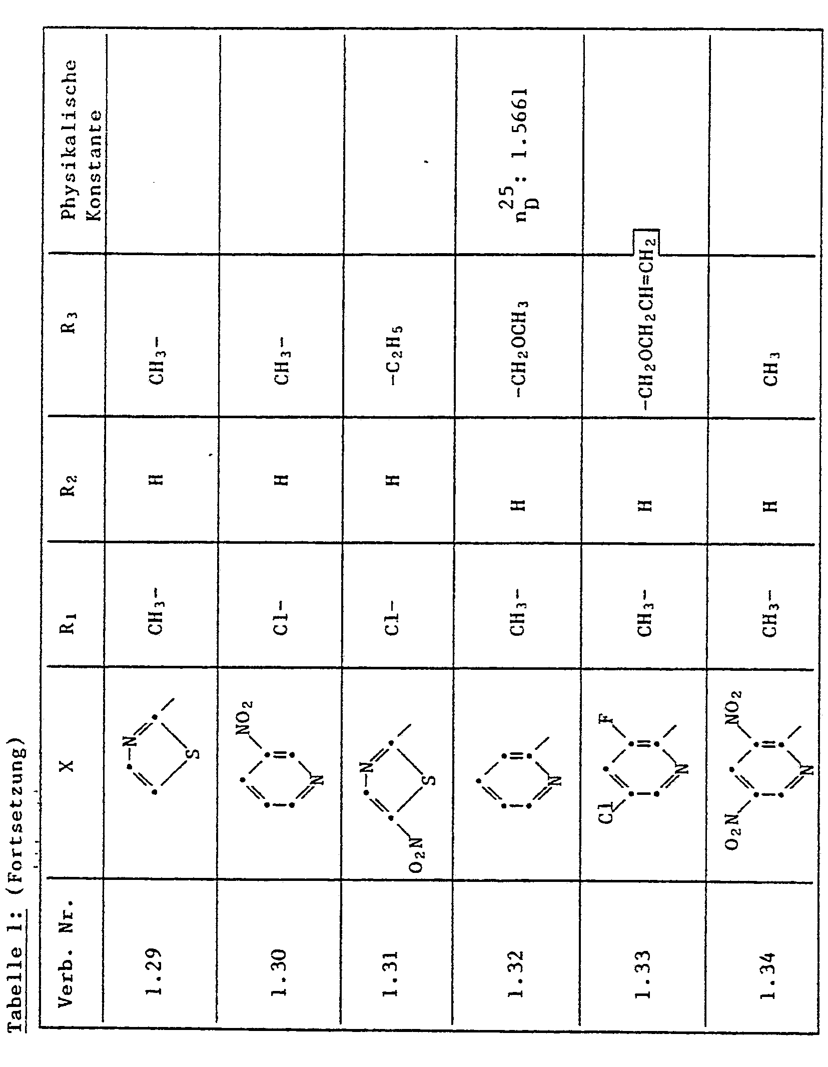

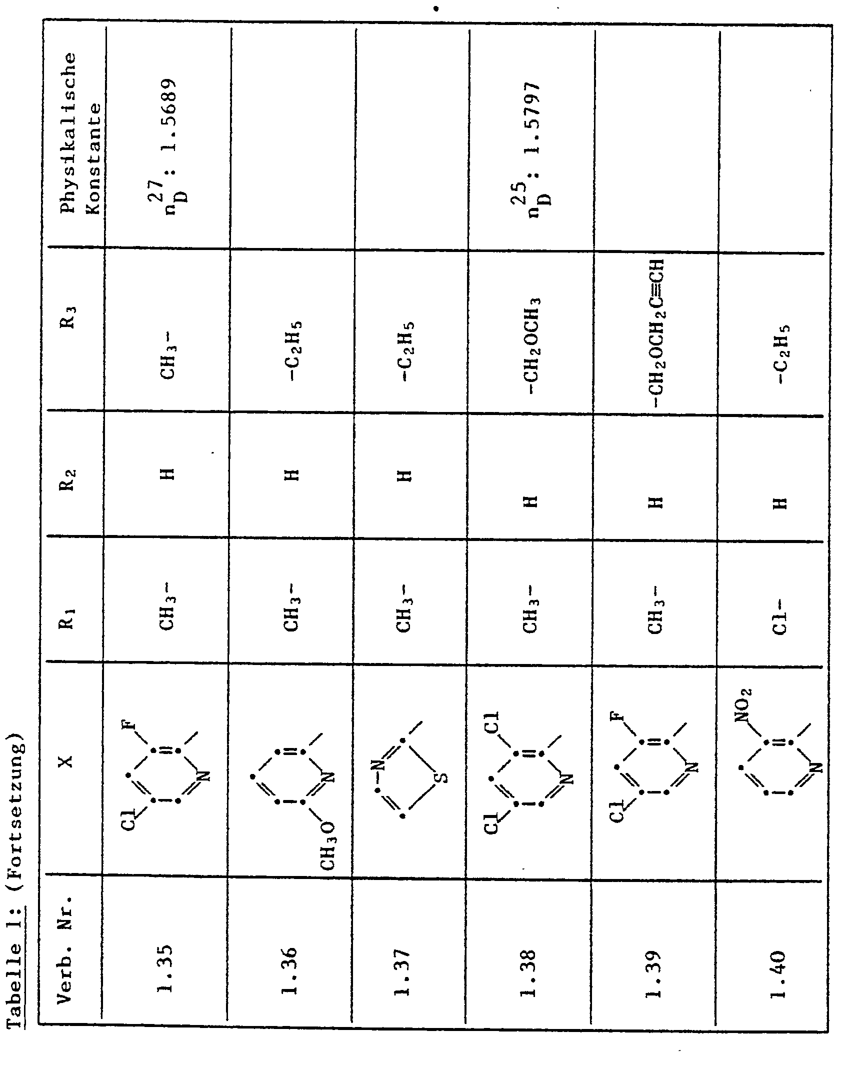

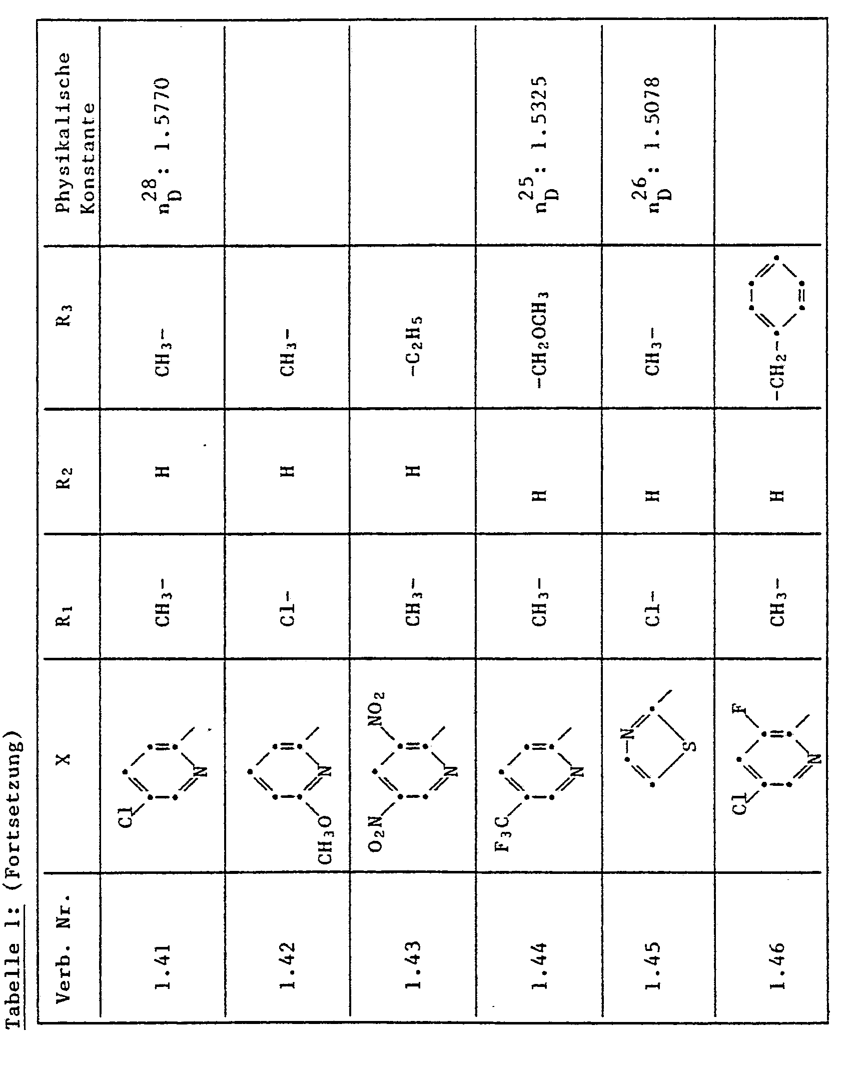

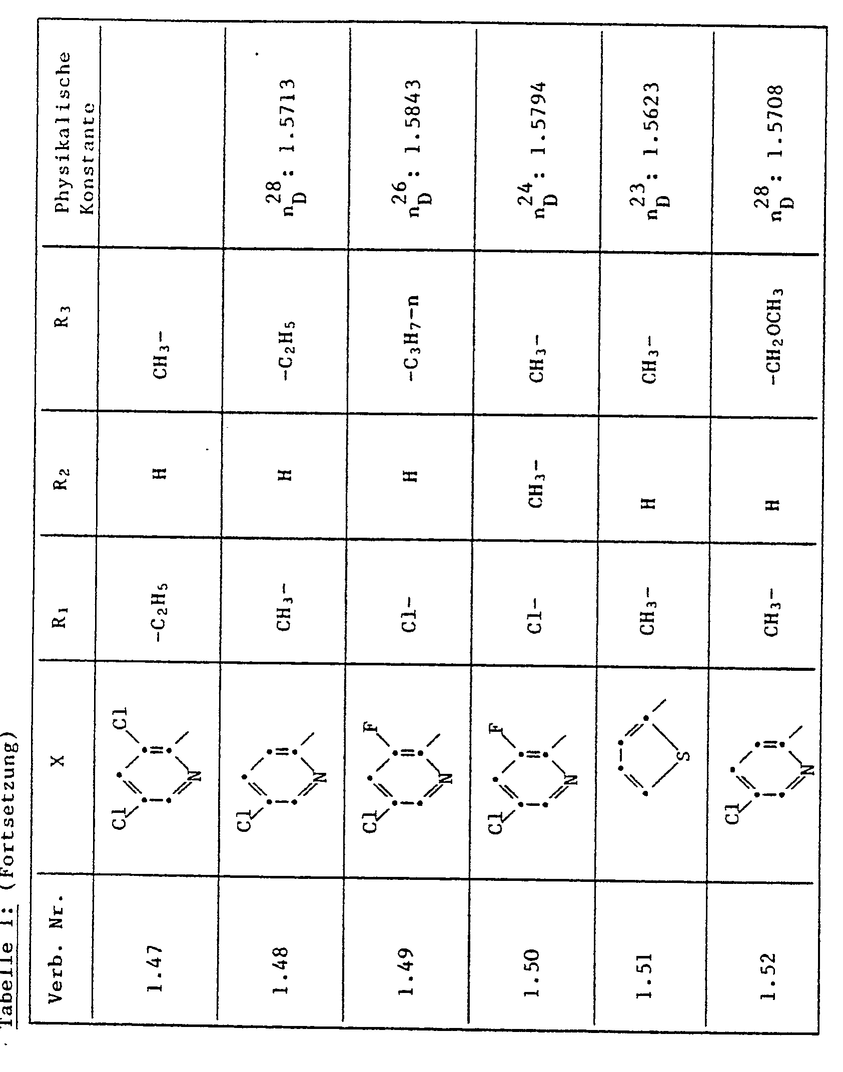

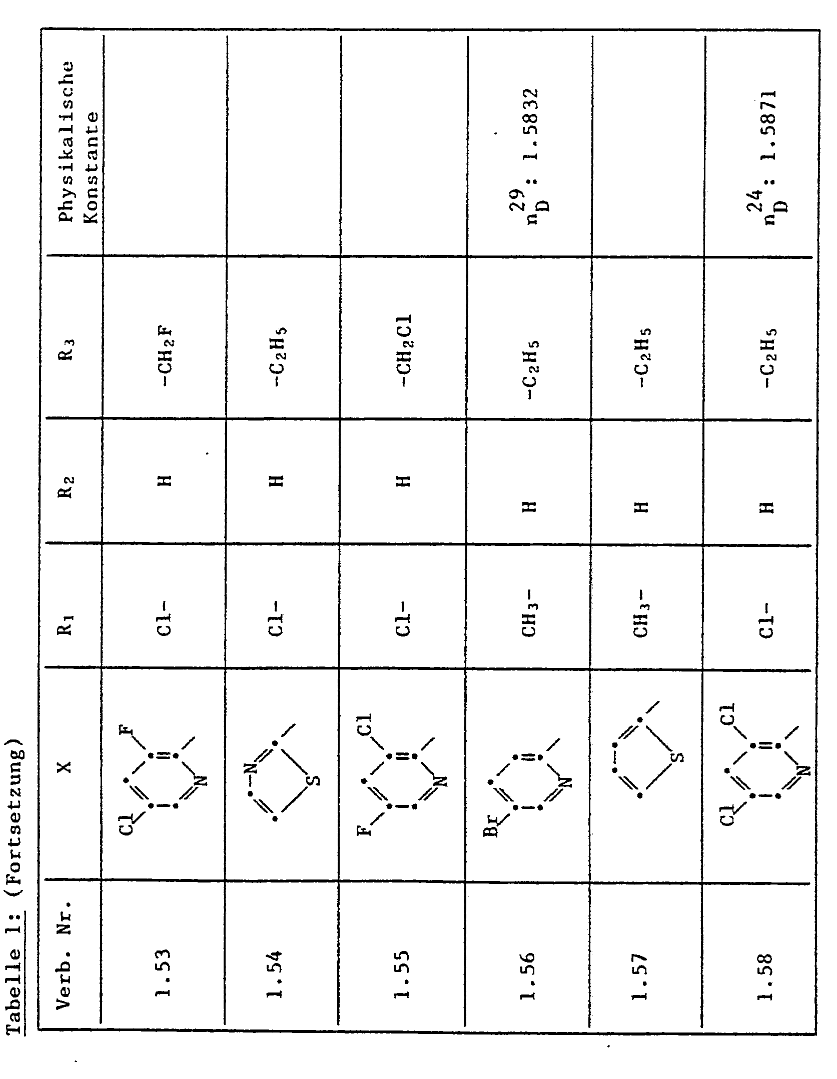

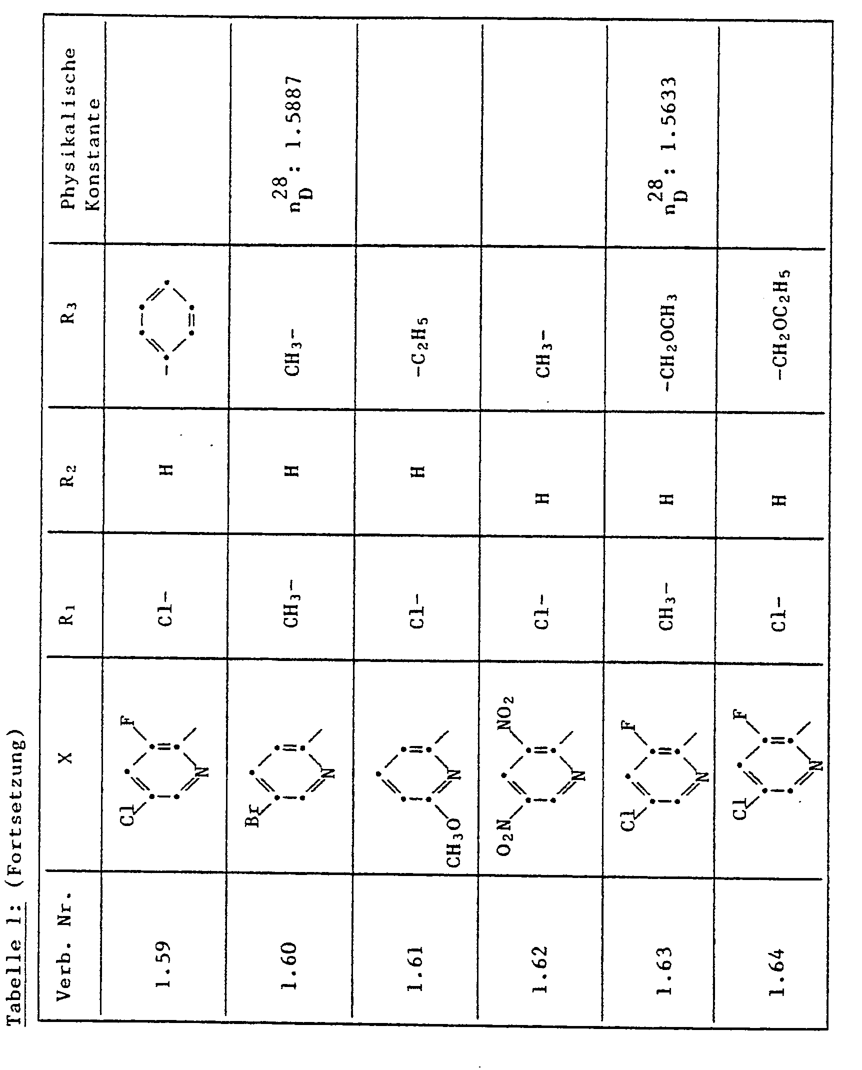

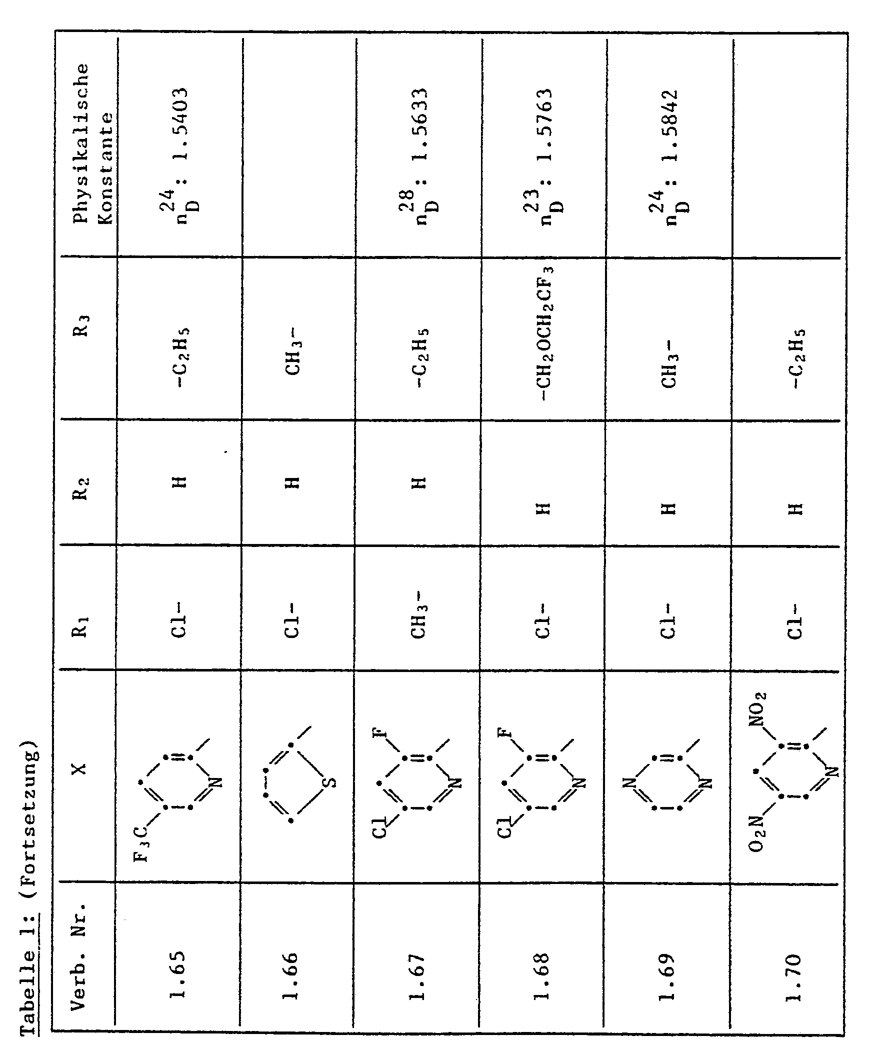

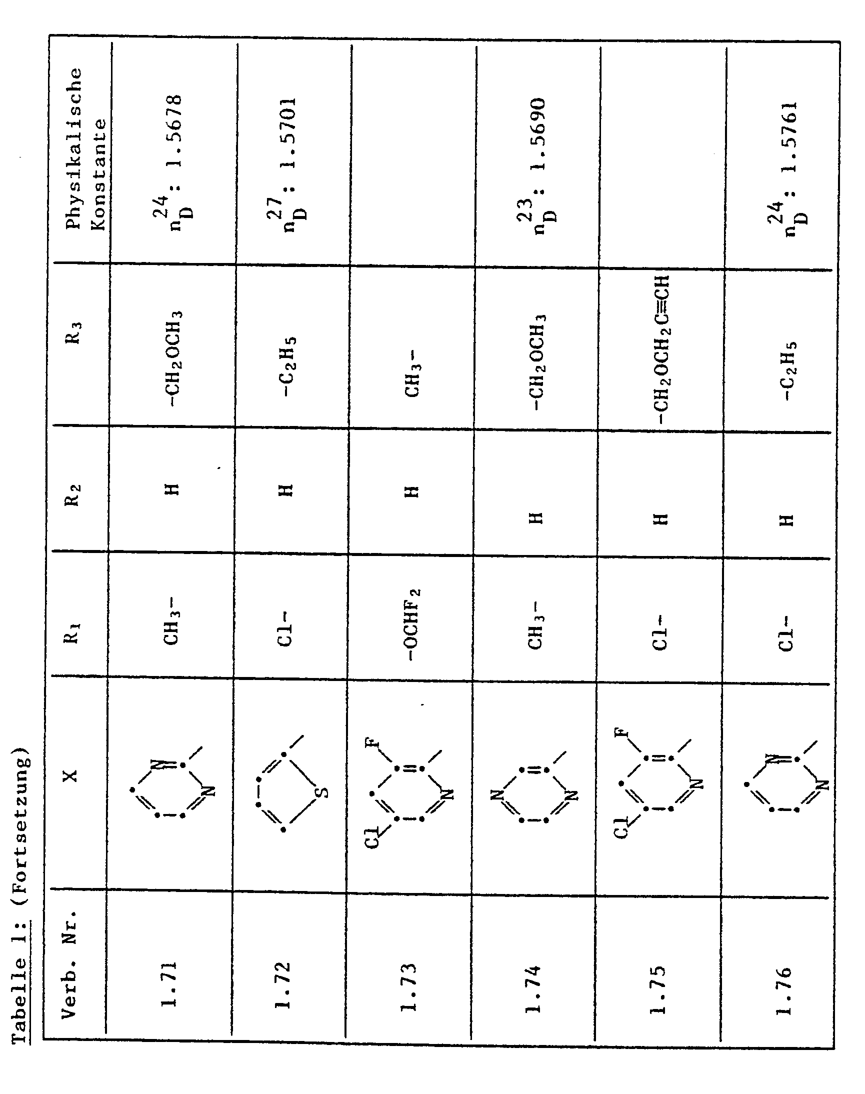

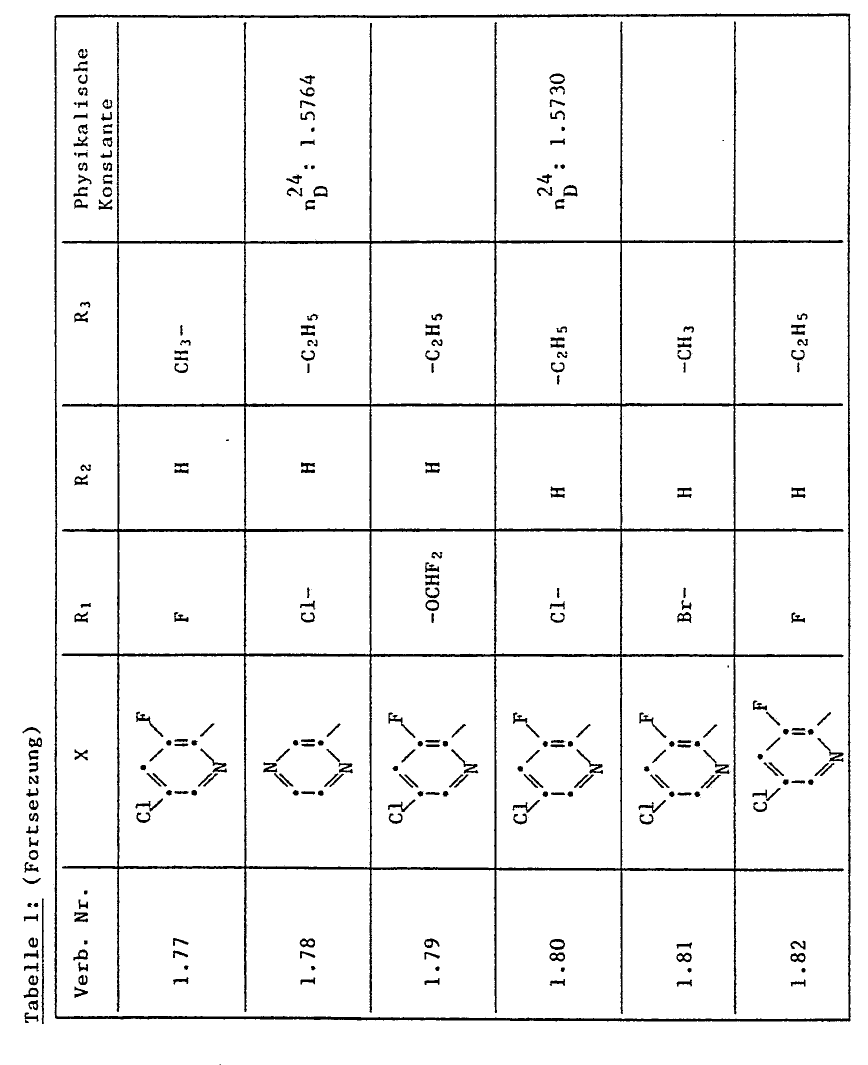

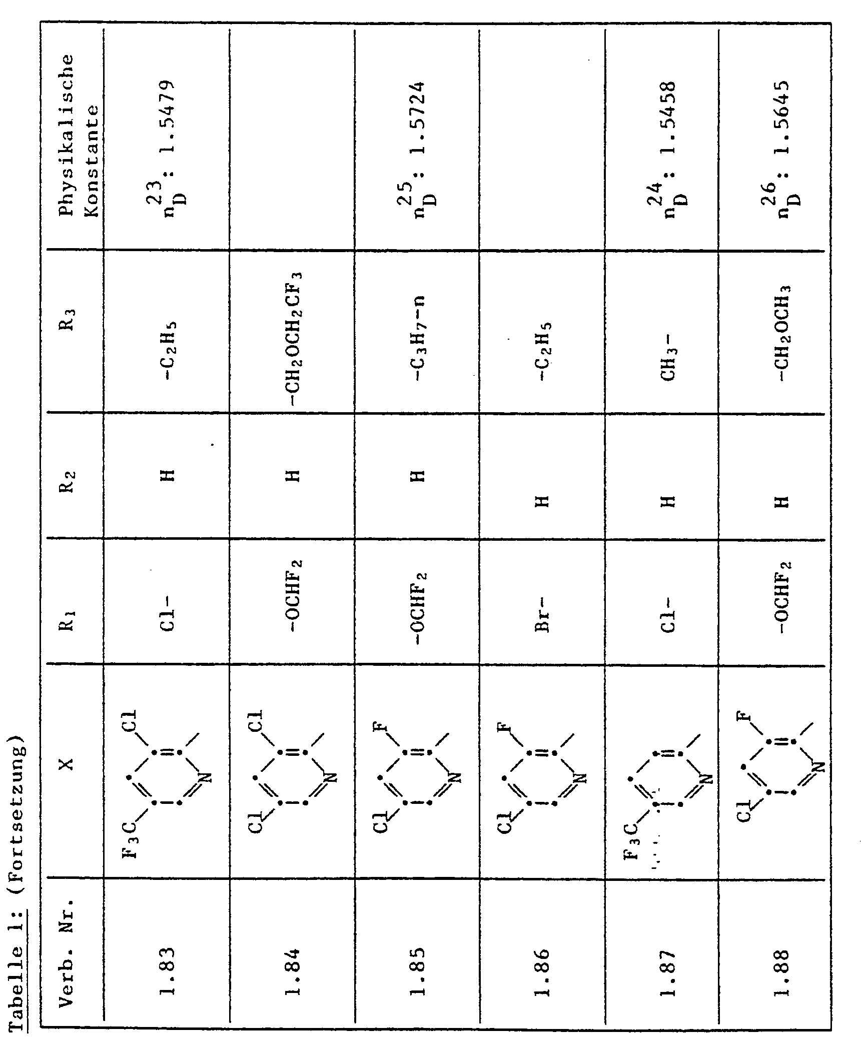

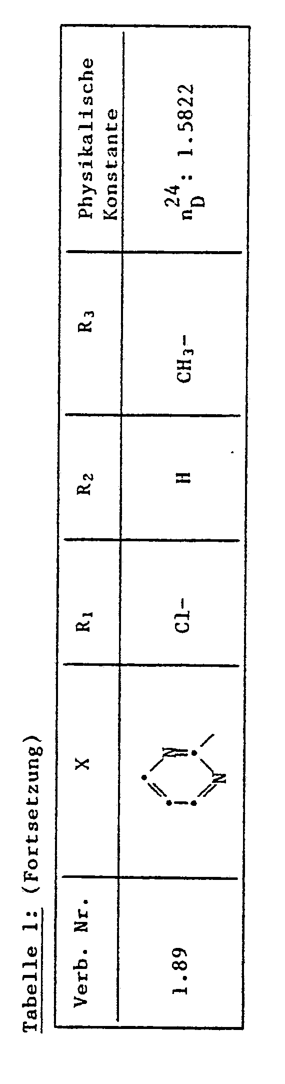

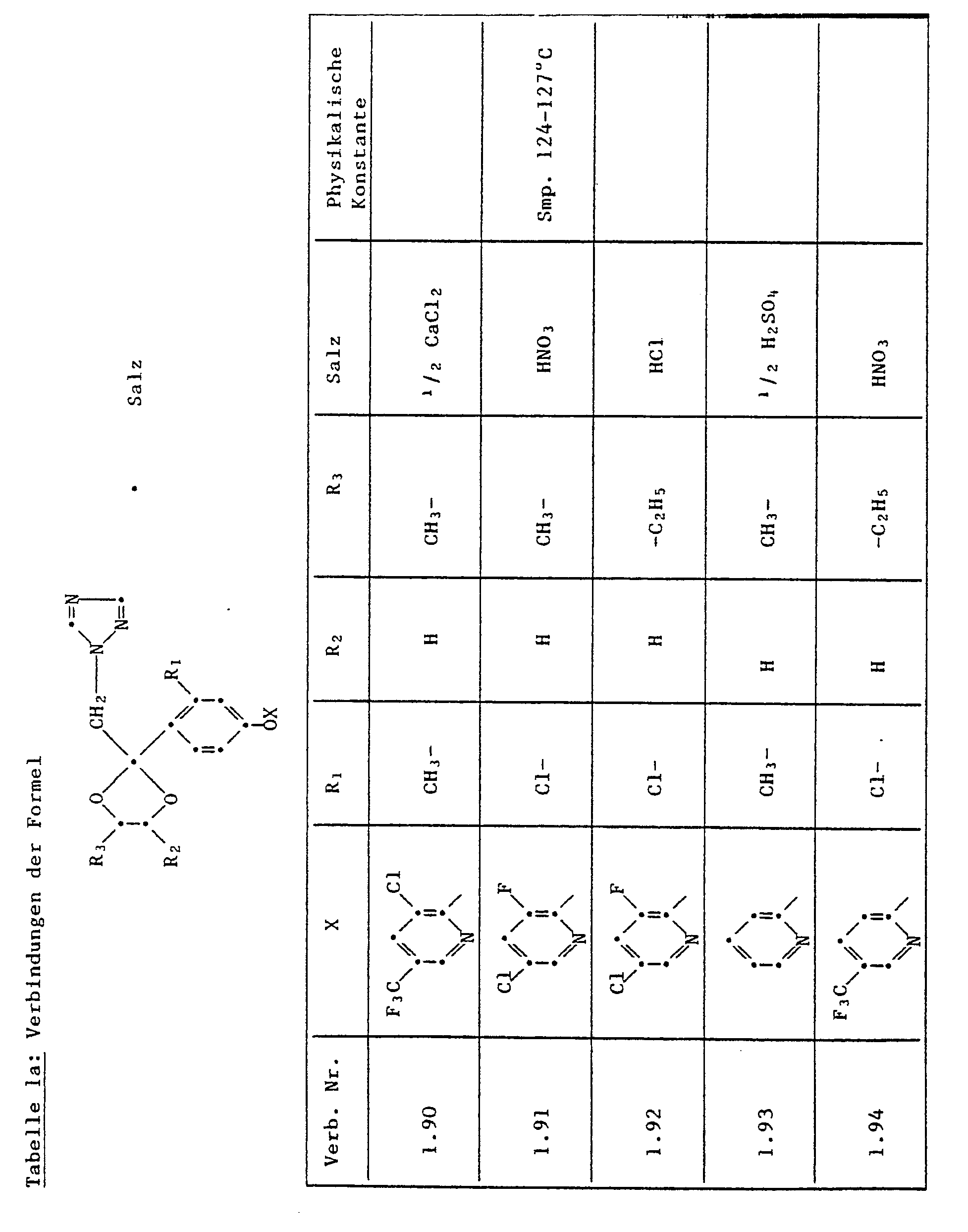

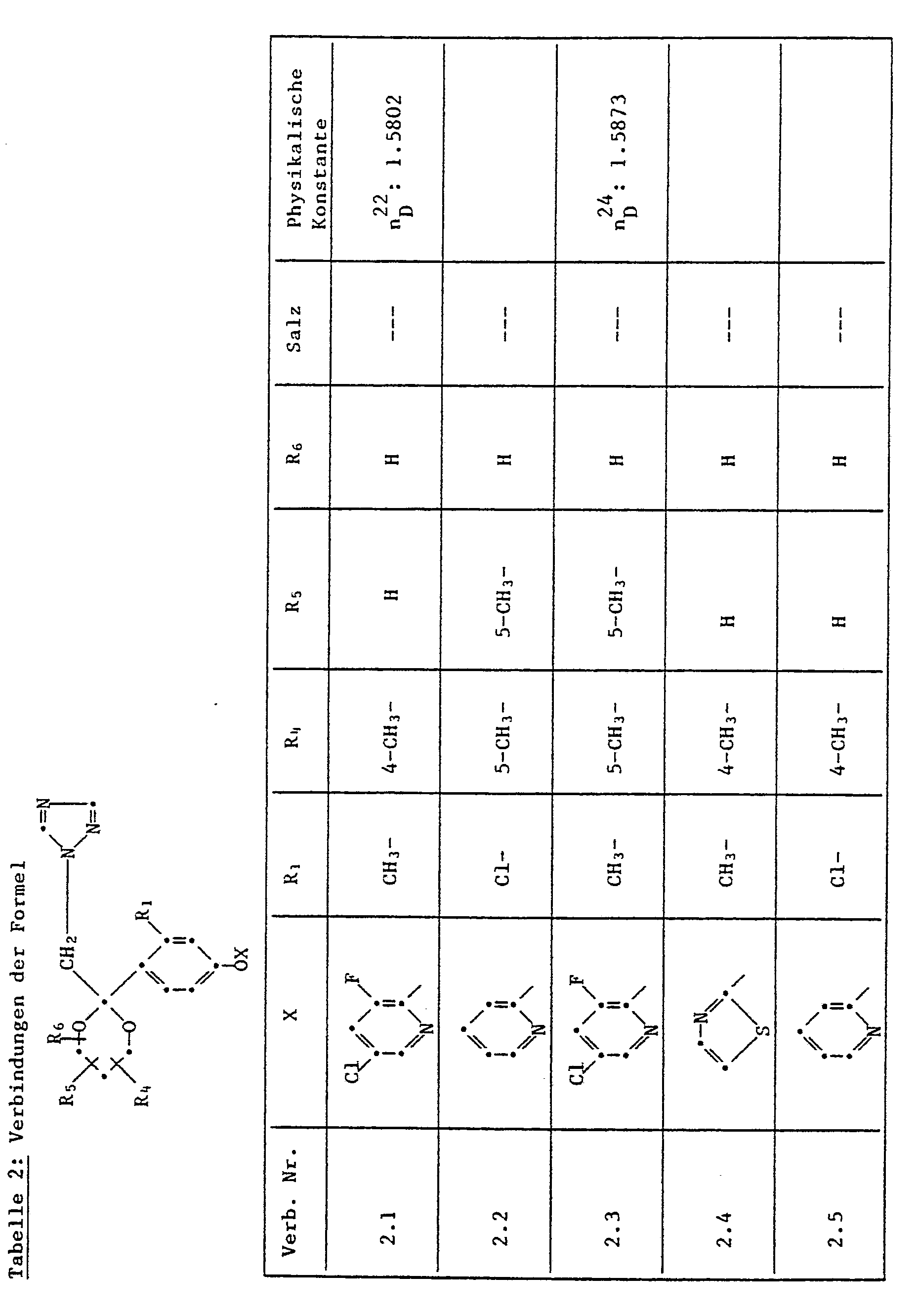

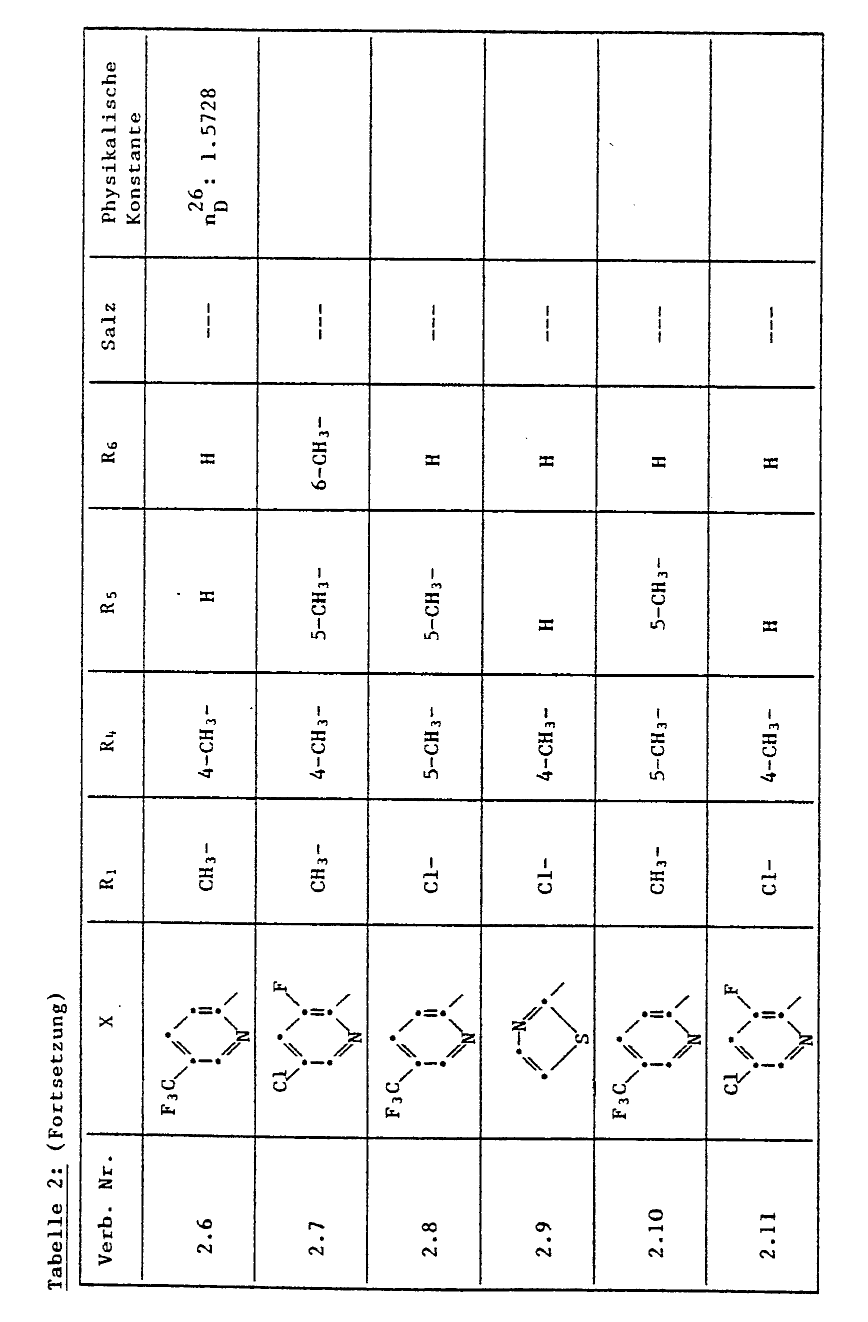

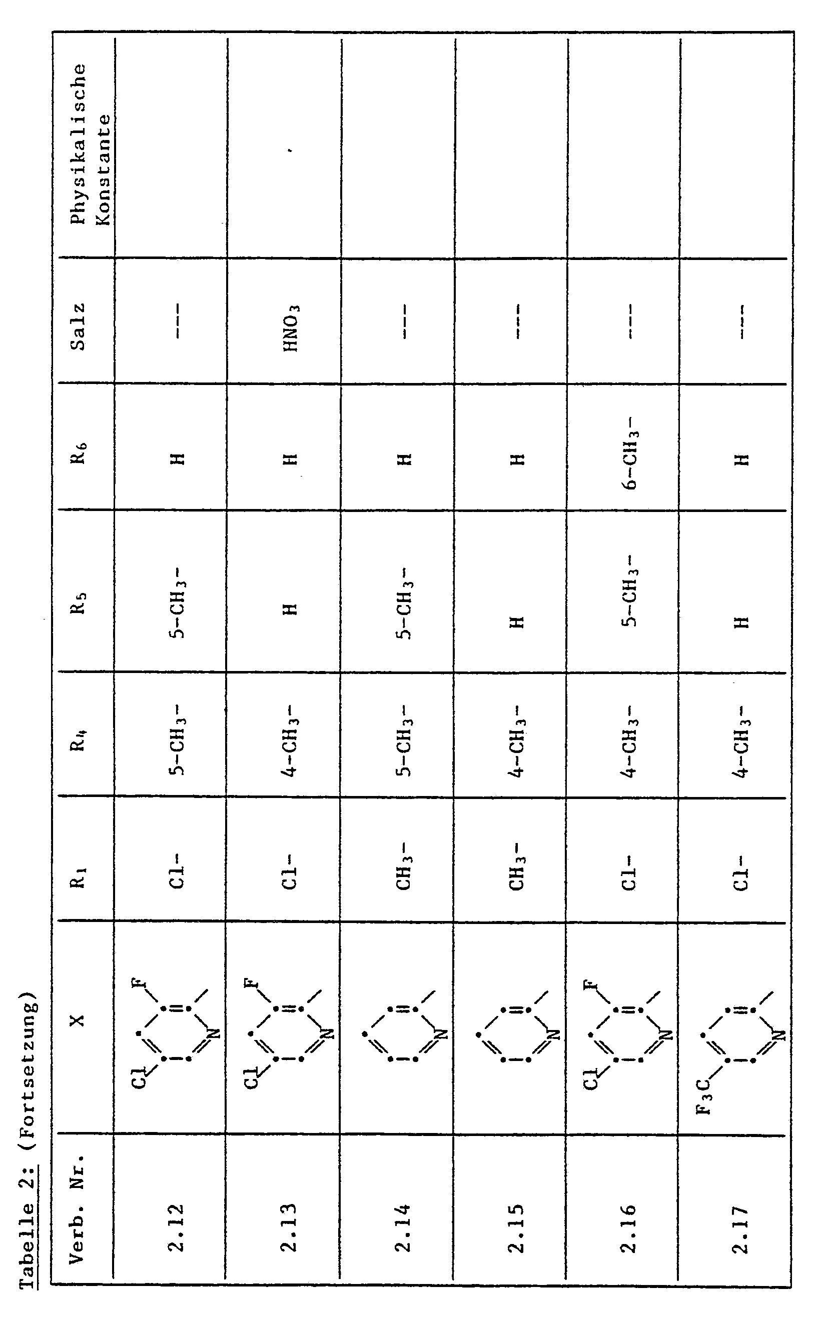

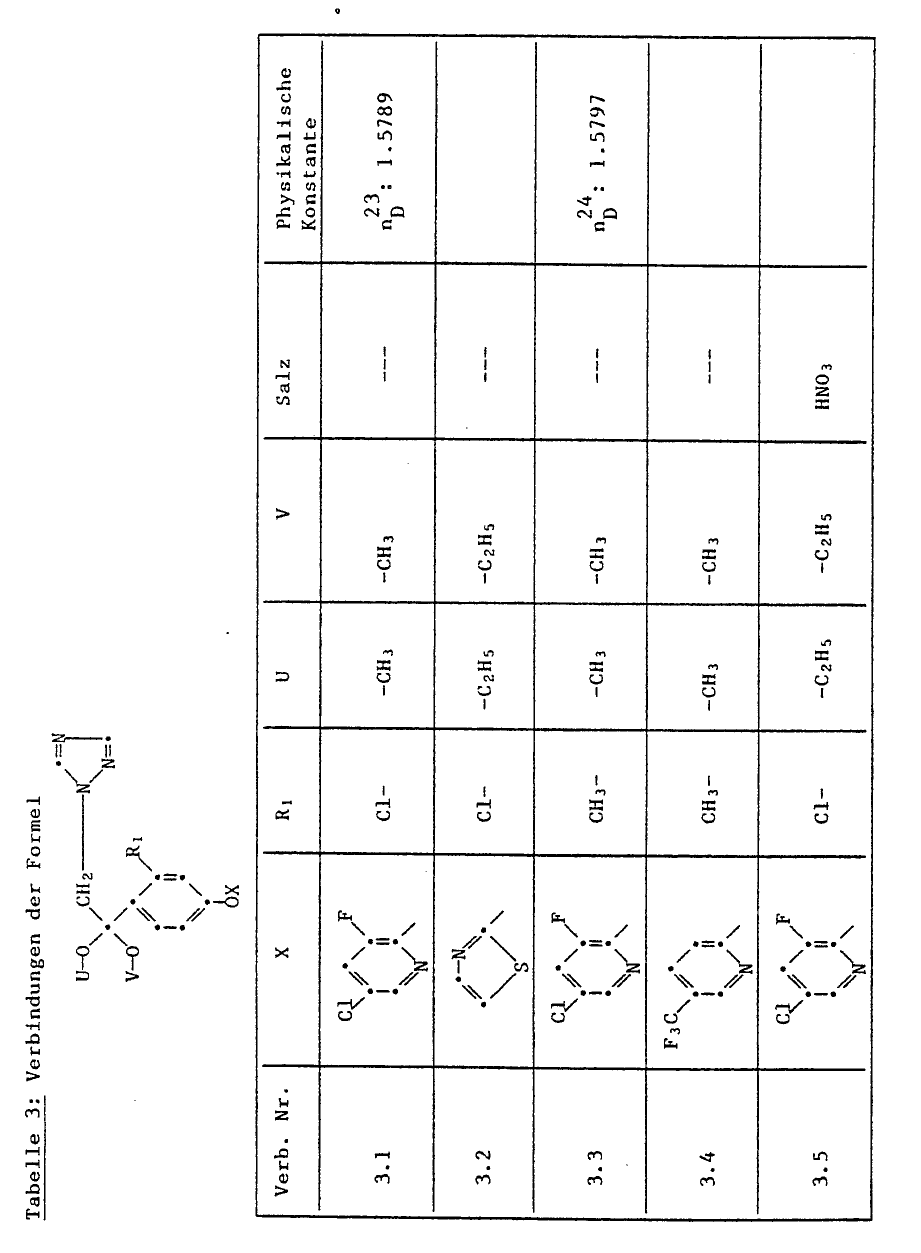

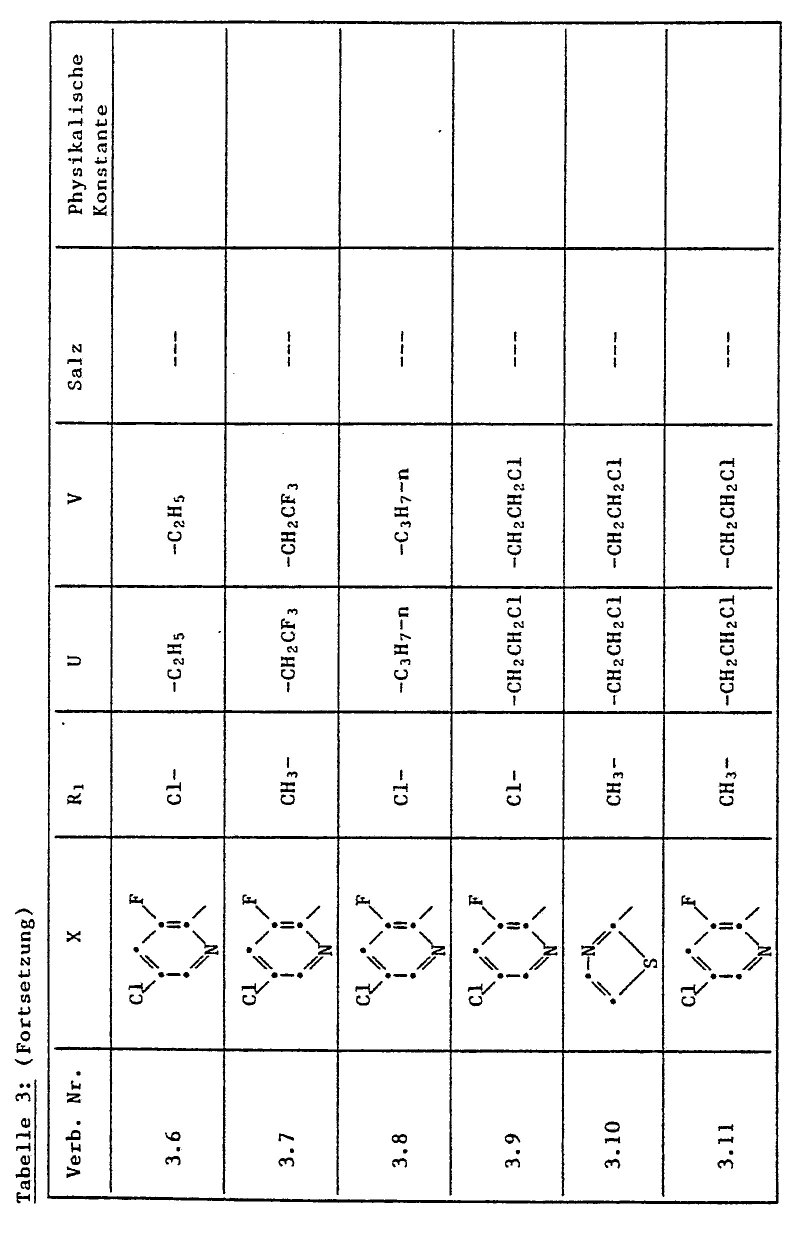

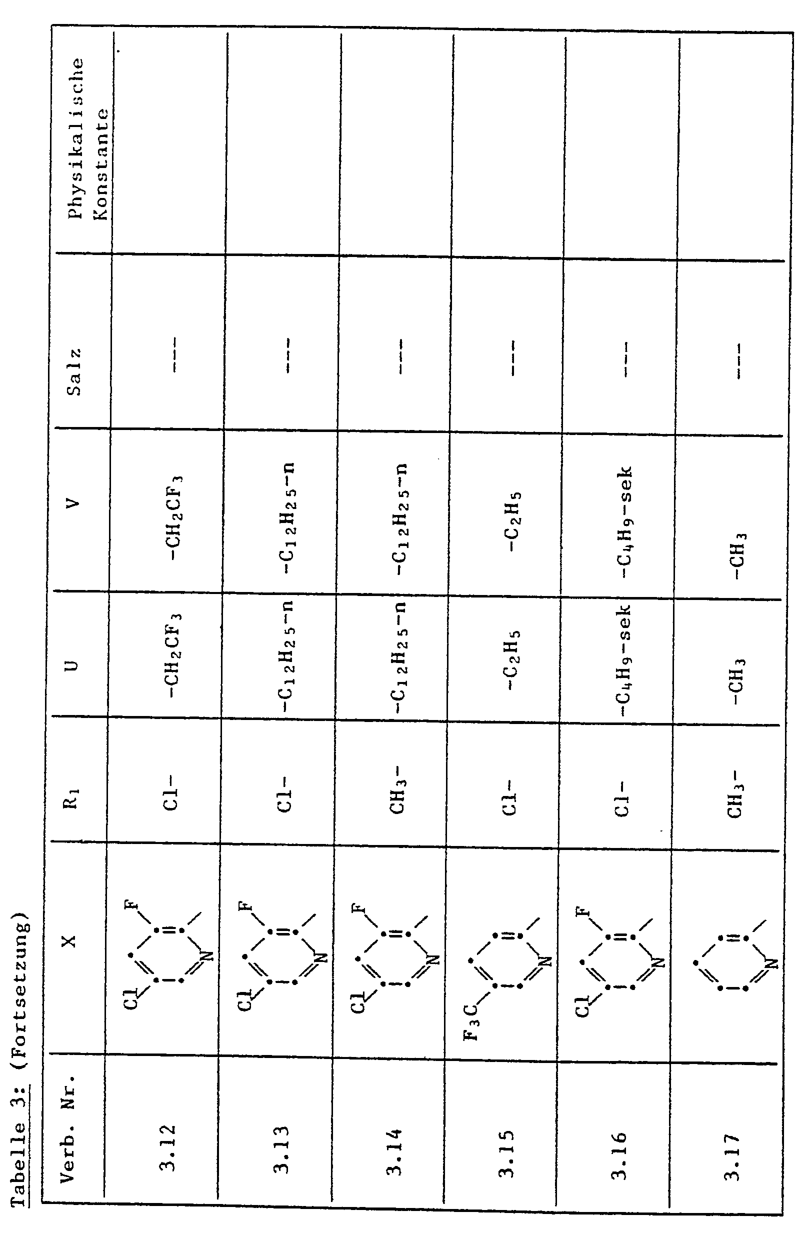

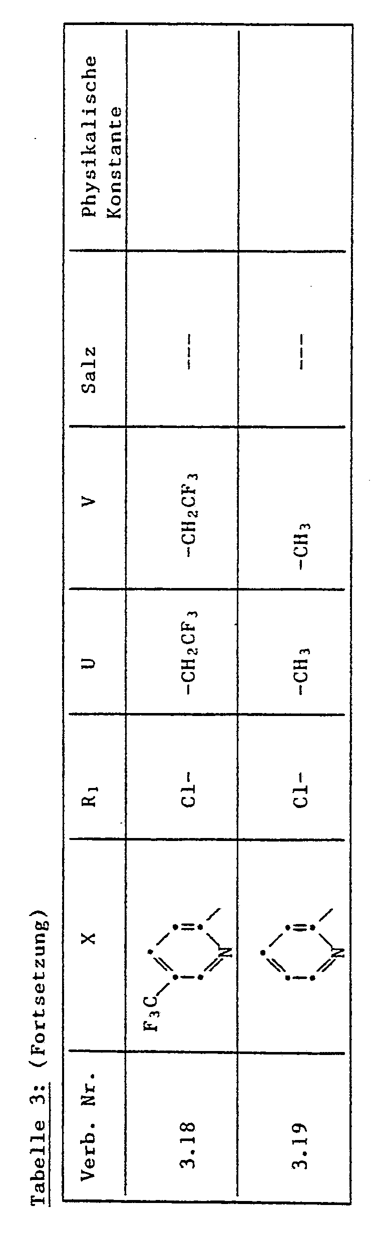

### Formulierungsbeispiele für flüssige Wirkstoffe der Formel I (% =Gewichtsprozent)

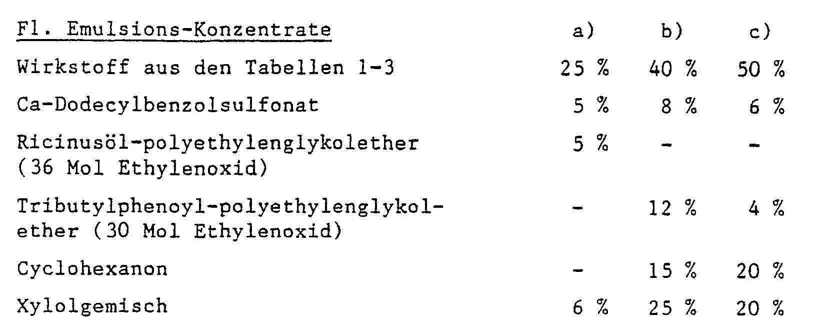

Aus solchen Konzentraten können durch Verdünnen mit Wasser Emulsionen jeder gewünschten Konzentration hergestellt werden.

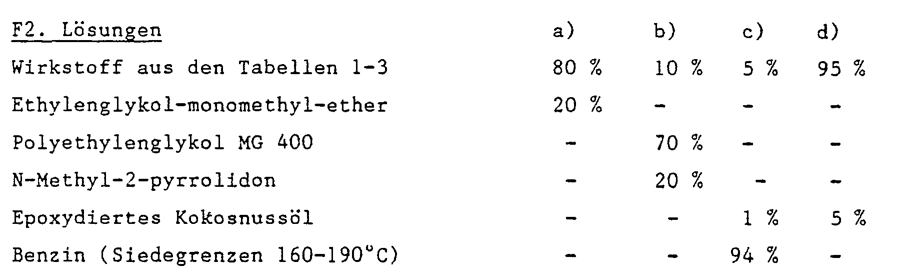

(MG = Molekulargewicht)

Die Lösungen sind zur Anwendung in Form kleinster Tropfen geeignet.

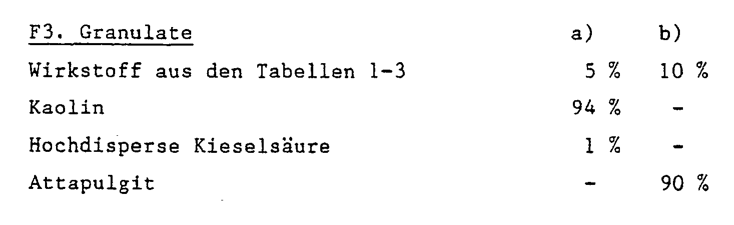

Der Wirkstoff wird in Methylenchlorid gelöst, auf den Träger aufgesprüht und das Lösungsmittel anschliessend im Vakuum abgedampft.

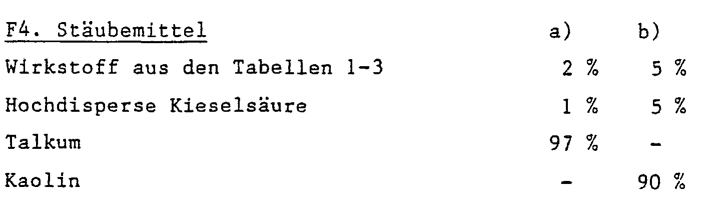

Durch inniges Vermischen auf Trägerstoffe mit dem Wirkstoff erhält man gebrauchsfertige Stäubemittel.

### Formulierungsbeispiele für feste Wirkstoffe der Formel 1 (% = Gewichtsprozent)

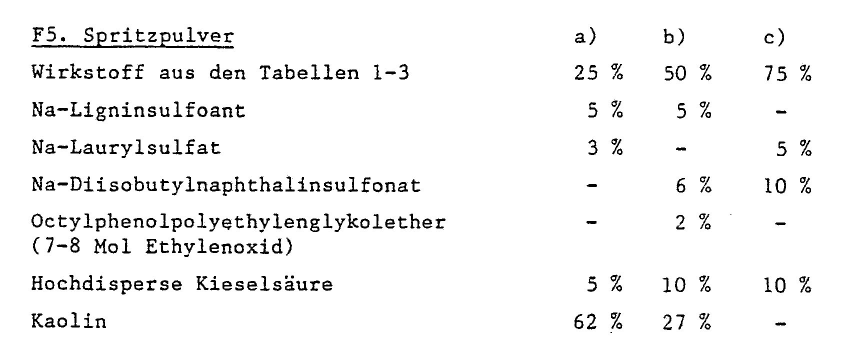

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

### F6. Emulsions-Konzentrat

Wirkstoff aus den Tabellen 1-3 10 %
Octylphenolpolyethylenglykolether (4-5 Mol Ethylenoxid) 5 %
Ca-Dodecylbenzolsulfonat 3 %
Ricinusölpolyglykolether (35 Mol Ethylenoxid) 4 %
Cyclohexanon 30 %
Xylolgemisch 50 %

Aus diesem Konzentrat können durch Verdünnen mit Wasser Emulsionen jeder gewünschten Konzentration hergestellt werden.

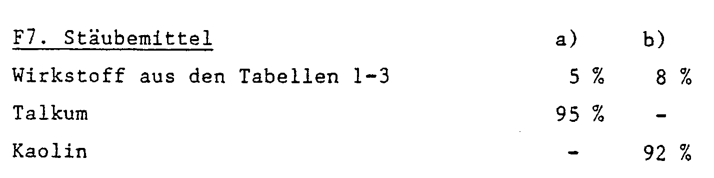

Man erhält anwendungsfertige Stäubemittel, indem der Wirkstoff mit dem Träger vermischt und auf einer geeigneten Mühle vermahlen wird.

### F8. Extruder-Granulat

Wirkstoff aus den Tabellen 1-3 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

### F9. Umhüllungs-Granulat

Wirkstoff aus den Tabellen 1-3 3 %
Polyethlyenglykol (MG 200) 3 %
Kaolin 94%
(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

### F10. Suspensions-Konzentrat

Wirkstoff aus den Tabellen 1-3 40 %
Ethlyenglykol 10%
Nonylphenolpolyethylenglykolether (15 Mol Ethylenoxid) 6 %
N-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele:

### Beispiel B1: Wirkung gegen Puccinia graminis auf Weizen

### a) Residual-protektive Wirkung

Weizenpflanzen wurden 6 Tage nach der Aussaat mit einer aus Spritzpulver des Wirkstoffes hergestellten Spritzbrühe (0,06 % Aktivsubstanz) besprüht. Nach 24 Stunden wurden die behandelten Pflanzen mit einer Uredosporensuspensionen des Pilzes infiziert. Nach einer Inkubation während 48 Stunden bei 95-100 % relativer Luftfeuchtigkeit und ca. 20 C wurden die infizierten Pflanzen in einem Gewächshaus bei ca. 22 C aufgestellt. Die Beurteilung der Rostpustelnentwicklung erfolgte 12 Tage nach der Infektion.

### b) Systemische Wirkung

Zu Weizenpflanzen wurde 5 Tage nach der Aussaat eine aus Spritzpulver des Wirkstoffs hergestellte Spritzbrühe gegossen (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 C wurden die infizierten Pflanzen in einem Gewächshaus bei ca. 22 C aufgestellt. Die Beurteilung der Rostpustelnentwicklung erfolgte 12 Tage nach der Infektion.

Verbindungen aus der Tabelle 1 zeigten gegen Puccinia-Pilze eine gute Wirkung. Unbehandelte aber infizierte Kontrollpflanzen zeigten einen Puccinia-Befall von 100 %. Unter anderem hemmten die Verbindungen Nr. 1.3, 1.6, 1.9, 1.20, 1.23, 1.27, 1.35, 1.48, 1.63 und 1.65 den Puccinia-Befall auf 0 bis 5 %. Darüberhinaus zeigten die Verbindungen Nr. 1.3 und 1.65 volle systemische Wirkung (0 % Befall) auch noch bei einer Verdünnung auf 0,006 %.

### Beispiel B2: Wirkung gegen Cercospora arachidicola auf Erdnusspflanzen

10-15 cm hohe Erdnusspflanzen wurden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus der Tabelle 1 behandelt wurden, einen stark reduzierten Cercospora-Befall. So verhinderten die Verbindungen Nr. 1.3, 1.5, 1.9, 1.12, 1.16, 1.20. 1.26, 1.27, 1.32, 1.35, 1.41, 1.44, 1.48, 1.52, 1.56, 1.60, 1.63, 1.65, 1.69, 1.74, 1.76, 1.78, 1.80 und 1.87 in obigen Versuchen das Auftreten von Flecken fast vollständig (0-10%).

### Beispiel B3: Wirkung gegen Erysiphae graminis auf Gerste

### a) Residual-protektive Wirkung

Ca. 8 cm hohe Gerstenpflanzen wurden mit einer aus Spritzpulver des Wirkstoffes hergestellten Spritzbrühe (0,02 Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 C aufgestellt und der Pilzbefall nach 10 Tagen beurteilt.

### b) Systemische Wirkung

Zu ca. 8 cm hohen Gerstenpflanzen wurde eine aus Spritzpulver des Wirkstoffes hergestellte Spritzbrühe gegossen (0,006 Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 C aufgestellt und der Pilzbefall nach 10 Tagen beurteilt.

Verbindungen der Formel I zeigten eine gute residual protektive Wirkung gegen Erysiphe-Pilze. Unbehandelte, aber infizierte Kontrollpflanzen zeigten einen Erysiphe-Befall von 100 %. Unter anderen Verbindungen aus der Tabelle 1 hemmten die Verbindungen Nr. 1.1, 1.5, 1.6, 1.9, 1.13, 1.16, 1.20, 1.23, 1.56, 1.63. 1.80 und 1.87 den Pilzbefall bei Gerste auf 0 bis 5 %. Darüberhinaus zeigten die Verbindungen 1.20 und 1.41 systemische Wirkung (0% Befall) bei einer Verdünnung auf 0,006 %.

### Beispiel B4: Residual-protektive Wirkung gegen Venturia inae ualis auf Apfeltrieben.

Apfelstecklinge mit 10-20 cm langen Frischtrieben wurden mit einer aus Spritzpulver des Wirkstoffes hergestellten Spritzbrühe (0,02 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24° C aufgestellt. Der Schorfbefall wurde 15 Tage nach der Infektion beurteilt. Verbindungen aus den Tabellen 1 und 2 bewirkten eine deutliche Hemmung des Krankheitsbefalls. So hemmten die Verbindungen Nr. 1.3, 1.5, 1.9, 1.12, 1.20, 1.23, 1.27, 1.32, 1.35, 1.38, 1.41, 1.44, 1.48, 1.52, 1.56, 1.60, 1.65, 1.69, 1.76, 1.78, 1.80 und 1.87 den Pilzbefall fast vollständig (0-5 %). Unbehandelte aber infizierte Triebe zeigten einen 100%igen Venturia-Befall.

### Beispiel B5: Wirkung gegen Botrytis cinerea auf Aepfeln Residual protektive Wirkung

Künstlich verletzte Aepfel werden behandelt, indem eine aus Spritzpulver der Wirksubstanz hergestellte Spritzbühe (0,02 % AS) auf die Verletzungsstellen aufgetropft wird. Die behandelten Früchte werden anschliessend mit einer Sporensuspension von Botrytis cinerea inokuliert und während einer Woche bei hoher Luftfeuchigkeit und ca. 20 C inkubiert.

Bei der Auswertung werden die pilzbefallenen Verletzungsstellen gezählt und daraus die fungizide Wirkung der Testsubstanz abgeleitet.

So vermindern z.B. die Verbindungen Nr. 1.3, 1.5, 1.48, 1.60, 1.63, 1.76 und 1.80 den Pilzbefall auf 0 bis 5 % (volle Wirkung).

## Patentansprüche

1. Verbindungen der Formel I 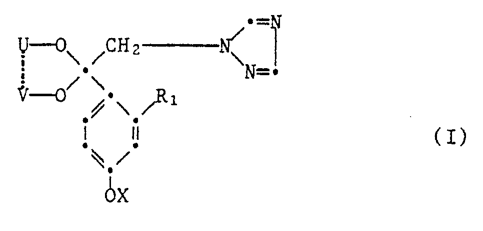 worin
X einen fünf- oder sechsgliedrigen ungesättigten heterocyclischen Rest mit einem, zwei oder drei Heteroatomen bedeutet, der unsubstituiert oder durch Halogen, Nitro, Ci-C₃-Alkyl, C₁-C₃-Alkoxy, C₁-C₃a-Halogenal-
koxy oder Trifluormethyl mono-, di- oder trisubstituiert sein kann,
R, Wasserstoff, Halogen, C₁-C₃-Alkyl oder C₁-C₃-Halogenalkoxy bedeutet und
U und V unabhängig voneinander gegebenenfalls durch Halogen oder Ci-C₆-Alkoxy substituiertes C₁-C₁₂-Alkyl darstellen oder zusammen folgende Alkylenbrücken 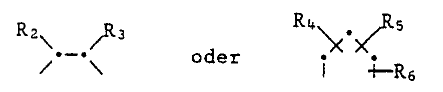 bilden, wobei
R₂ und R₃ unabhängig voneinander für Wasserstoff, C₁-C₆-Alkyl, ein-oder mehrfach durch Halogen substituiertes C₁-C₆-Alkyl, Phenyl, ein-oder mehrfach durch Halogen und/oder Ci-C₂-Alkyl substituiertes Phenyl oder die Gruppe -CH₂-Z-R₇ stehen oder worin R₂ und R₃ zusammen eine unsubstituierte oder durch C₁-C₄-Alkyl substituierte Tetramethylenbrücke bilden; und wobei
Z Sauerstoff oder Schwefel bedeutet;
R₇ für Wasserstoff, C₁-C₆-Alkyl, ein- oder mehrfach durch Halogen oder C₁-C₃-Alkoxy substituiertes C₁-C₆-Alkyl, oder für C₃-C₄-Alkenyl, 2-Propinyl, 3-Halo-2-propinyl, oder auch Phenyl oder Benzyl steht, deren aromatischer Ring unsubstituiert oder durch Halogen, C₁-C₃-Alkyl, C₁-C₃-Alkoxy, Nitro, und/oder Trifluormethyl ein- oder mehrfach substituiert ist;
R₄, Rs und R₆ unabhängig voneinander Wasserstoff oder C₁-C₄-Alkyl bedeuten, wobei die Gesamtzahl der Kohlenstoffatome in R₄, Rs und R₆ die Zahl 6 nicht übersteigt;
unter Einschluss ihrer Säureadditionssalze und Metallkomplexe.

2. Verbindungen gemäss Anspruch 1, dadurch gekennzeichnet, dass X einen unsubstituierten oder durch Methoxy, Halogen, Nitro oder Trifluormethyl mono- oder disubstituierten 2-Pyridinylrest, den 2-Thienylrest, den 2-Thiazolylrest oder den 4-Nitro-2-thiazolylrest, den Pyrazinylrest, den 2-Pyrimidinylrest oder den 4,5,6-Trichlor-2-pyrimidinylrest,
R₁ Wasserstoff, Halogen, Methyl, Aethyl oder Difluormethoxy bedeuten und U und V unabhängig voneinander unsubstituierte oder durch Chlor oder Trifluormethyl substituierte C₁-C₁₂-Alkyl darstellen oder zusammen folgende Alkylenbrücken 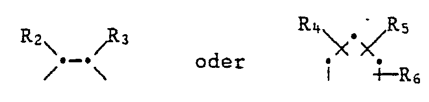 bilden, wobei
R₂ und R₃ unabhängig voneinander für Wasserstoff, Phenyl, durch Halogen substituiertes Phenyl, unsubstituiertes oder durch Halogen, Cₗ-C₃-Alkoxy, C₁-C₂-Halogenalkoxy, Allyloxy, Propargyloxy, substituiertes C₁-C₃-Alkyl stehen.

3. Verbindungen gemäss Anspruch 2, dadurch gekennzeichnet, dass X einen unsubstituierten oder durch Fluor, Chlor, Brom, Nitro oder Trifluormethyl mono- oder disubstituierten 2-Pyridinylrest, den 2-Thienylrest, den 2-Thiazolylrest, den Pyrazinylrest, den 2-Pyrimidinylrest oder den 4,5,6-Trichlor-2-pyrimidinylrest,
R, Wasserstoff, Chlor, Methyl oder Difluormethoxy bedeuten und U und V Methyl, Aethyl, Propargyl darstellen oder zusammen folgende Alkylenbrücken 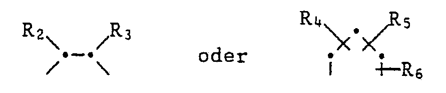 bilden, wobei
R₂ und R₃ unabhängig voneinander für Wasserstoff, Ci-C₃-Alkyl, Methoxymethyl, Aethoxymethyl oder - Trifluoraethoxymethyl, R₄ für Methyl, Rₛ für Wasserstoff oder Methyl und R₆ für Wasserstoff stehen.

4. Verbindungen gemäss Anspruch 3, dadurch gekennzeichnet, dass X einen unsubstituierten oder einen durch Chlor, Brom oder Trifluormethyl mono- oder disubstituierten 2-Pyridinylrest oder den 2-Pyrazinylrest,
R, Methyl oder Chlor,
U und V zusammen den Methyl-äthylenrest, den Aethyl-äthylenrest oder den n-Propyl-äthylenrest bedeuten.

5. Verfahren zur Herstellung der in Anspruch 1 definierten Verbindungen der Formel I, dadurch gekennzeichnet, dass man A) Verbindungen der Formeln II und III 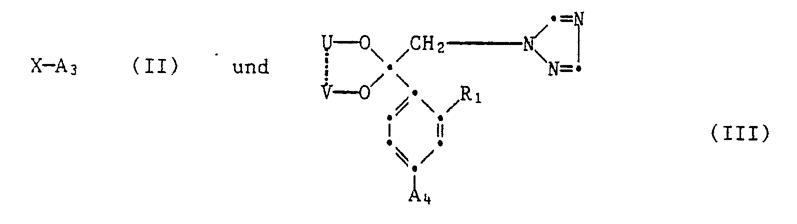 worin einer der Reste A₃ und A₄ eine Gruppe -O-Me, in der Me Wasserstoff oder vorzugsweise ein Metallkation ist, und der andere einen, gegen einen definitionsgemäss substituierten Hydroxylrest austauschbaren Rest bedeutet, miteinander kondensiert oder
B) eine Verbindung der Formel IV 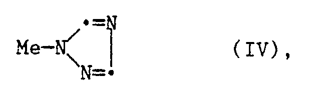 worin Me Wasserstoff oder ein Metallkation darstellt, mit einer Verbindung der Formel V 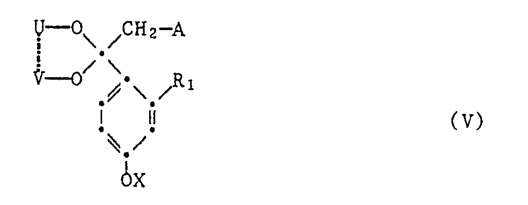 worin A eine nukleofuge Abgangsgruppe bedeutet, kondensiert oder
C) in einer Verbindung der Formel VI 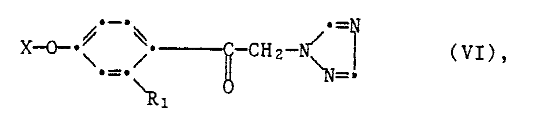 die Carbonylgruppe in eine Gruppe der Formel VII 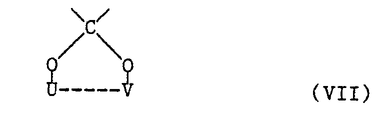 überführt, oder
D) zur Herstellung von Verbindungen der Formel I, worin U und V gemeinsam eine Gruppe der Formel -CH₂-CH(CH₂ZR )- darstellen und R einen von Wasserstoff verschiedenen Rest R₇ bedeutet, Verbindungen der Formeln VIII und IX 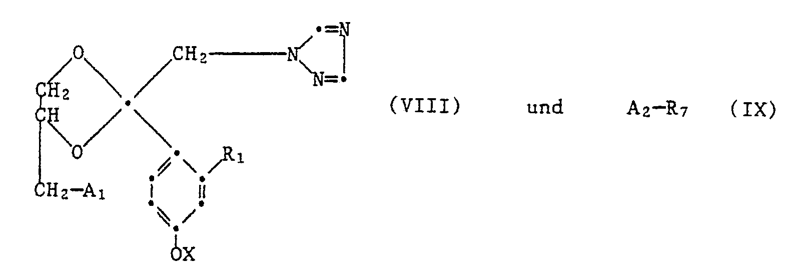 worin einer der Reste A, und A₂ gegebenenfalls in Salzform vorliegendes Hydroxy oder Mercapto, z.B. der Formel -Z-Me, und der andere eine nukleofuge Abgangsgruppe A bedeutet oder sowohl A, als auch A₂ Hydroxygruppen darstellen, miteinander kondensiert, und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhältliche freie Verbindung bzw. ein verfahrensgemäss erhältliches Säureadditionssalz in einen Metallkomplex überführt.

6. Verfahren nach Anspruch 5, dadurch gekennzeichnet, dass man eine Verbindung der Formel gemäss einem der Ansprüche 1-4 herstellt.

7. Verbindungen gemäss einem der Ansprüche 1-4, zur Behandlung von Pflanzen.

8. Agrochemisches Schädlingsbekämpfungsmittel zur Verhütung eines Befalls durch Mikroorganismen, dadurch gekennzeichnet, dass es als mindestens eine aktive Komponente eine Verbindung der Formel I gemäss Anspruch 1 enthält.

9. Mittel gemäss Anspruch 8, dadurch gekennzeichnet, dass es als mindestens eine aktive Komponente eine Verbindung der Formel I gemäss einem der Ansprüche 2 bis 4 enthält.

10. Mittel nach einem der Ansprüche 8 oder 9, dadurch gekennzeichnet, dass es 0,1 bis 99% eines Wirkstoffs der Formel I, 99,9 % bis 1 % eines festen oder flüssigen Zusatzstoffes und 0 bis 25 % eines Tensides enthält.

11. Mittel nach Anspruch 10, dadurch gekennzeichnet, dass es 0,1 bis 95% eines Wirkstoffes der Formel I, 99,8 % bis 5 % eines festen oder flüssigen Zusatzstoffes und 0,1 bis 25 % eines Tensides enthält.

12. Verfahren zur Herstellung eines agrochemischen Mittels, dadurch gekennzeichnet, dass mindestens eine Verbindung der Formel I gemäss einem der Ansprüche 1 bis 4 mit geeigneten festen oder flüssigen Zusatzstoffen und Tensiden innig vermischt wird.

13. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 4 auf die Pflanze oder deren Standort appliziert.

14. Verwendung von Verbindungen der Formel I gemäss Anspruch 1 zur Bekämpfung und/oder zur präventiven Verhütung eines Befalls von Mikroorganismen an Pflanzen.

15. Verwendung nach Anspruch 14 von Verbindungen der Formel I gemäss einem der Ansprüche 2 bis 4.

16. Verwendung gemäss Anspruch 14, dadurch gekennzeichnet, dass es sich bei den Mikroorganismen um phytopathogene Pilze handelt.
